# EUROPÄISCHE PATENTANMELDUNG

(11) **EP 1 801 205 A2**
(43) Veröffentlichungstag der Anmeldung: **27.06.2007**
(21) Anmeldenummer: 07103392.2
(22) Anmeldetag: 31.10.2002
(51) Int. Cl.: C12N 9/12, C12N 9/00, C12N 15/54, C12N 15/31, C12N 15/77, C12P 1/04

(54) **Gene, die für Phosphoenolpyruvat-Zucker-Phosphotransferase Proteine codieren**

(30) Priorität: 05.11.2001 DE 10154276
(62) Teilanmeldung aus: 02783045.4
(71) Anmelder: BASF Aktiengesellschaft, 67056 Ludwigshafen (DE)
(72) Erfinder: Zelder, Oskar, 67346 Speyer (DE); Pompejus, Markus, Seoul 140-210 (KR); Schröder, Hartwig, 69226 Nußloch (DE); Kröger, Burkhard, 67117 Limburgerhof (DE); Klopprogge, Corinna, 68239 Mannheim (DE); Haberhauer, Gregor, 67117, Limburgerhof (DE)

(57) **Zusammenfassung**

Die Erfindung betrifft neue Nukleinsäuremoleküle, deren Verwendung zur Konstruktion von gentechnisch verbesserten Mikroorganismen und Verfahren zur Herstellung von Feinchemikalien, insbesondere Aminosäuren mit Hilfe dieser gentechnisch verbesserten Mikroorganismen.

SEQUENCE LISTING
<110> Zelder, Oskar
Pompejus, Markus
Schroder, Hartwig
Kroger, Burkhard
Klopprogge, Corinna
Haberhauer, Gregor

<120> Genes coding for phosphoenolpyruvate sugar
phosphotransferase proteins

<130> BGI-165US
<160> 8

<150> PCT/EP02/12140
<151> 2002-10-31

<150> DE 10154276
<151> 2001-11-05
<210> 1
<211> 1834
<212> DNA
<213> Corynebacterium glutamicum
<220>
<221> CDS
<222> (101)..(1804)
<223> RXA01244
<400> 1
gatatgtgtt tgtttgtcaa tatccaaatg tttgaatagt tgcacaactg ttggttttgt 60
ggtgatcttg aggaaattaa ctcaatgatt gtgaggatgg gtg gct act gtg gct 115
Val Ala Thr Val Ala
1               5
gat gtg aat caa gac act gta ctg aag ggc acc ggc gtt gtc ggt gga 163
Asp Val Asn Gln Asp Thr Val Leu Lys Gly Thr Gly Val Val Gly Gly
                10                  15                  20
gtc cgt tat gca agc gcg gtg tgg att acc cca cgc ccc gaa cta ccc 211
Val Arg Tyr Ala Ser Ala Val Trp Ile Thr Pro Arg Pro Glu Leu Pro
            25                  30                  35
caa gca ggc gaa gtc gtc gcc gaa gaa aac cgt gaa gca gag cag gag 259
Gln Ala Gly Glu Val Val Ala Glu Glu Asn Arg Glu Ala Glu Gln Glu
        40                  45                  50
cgt ttc gac gcc gct gca gcc aca gtc tct tct cgt ttg ctt gag cgc 307
Arg Phe Asp Ala Ala Ala Ala Thr Val Ser Ser Arg Leu Leu Glu Arg
    55                  60                  65
tcc gaa gct gct gaa gga cca gca gct gag gtg ctt aaa gct act gct 355
Ser Glu Ala Ala Glu Gly Pro Ala Ala Glu Val Leu Lys Ala Thr Ala
70                  75                  80                  85
ggc atg gtc aat gac cgt ggc tgg cgt aag gct gtc atc aag ggt gtc 403
Gly Met Val Asn Asp Arg Gly Trp Arg Lys Ala Val Ile Lys Gly Val
                90                  95                  100
aag ggt ggt cac cct gcg gaa tac gcc gtg gtt gca gca aca acc aag 451
Lys Gly Gly His Pro Ala Glu Tyr Ala Val Val Ala Ala Thr Thr Lys
            105                 110                 115
ttc atc tcc atg ttc gaa gcc gca ggc ggc ctg atc gcg gag cgc acc 499
Phe Ile Ser Met Phe Glu Ala Ala Gly Gly Leu Ile Ala Glu Arg Thr
        120                 125                 130
aca gac ttg cgc gac atc cgc gac cgc gtc atc gca gaa ctt cgt ggc 547
Thr Asp Leu Arg Asp Ile Arg Asp Arg Val Ile Ala Glu Leu Arg Gly
    135                 140                 145
gat gaa gag cca ggt ctg cca gct gtt tcc gga cag gtc att ctc ttt 595
Asp Glu Glu Pro Gly Leu Pro Ala Val Ser Gly Gln Val Ile Leu Phe
150                 155                 160                 165
gca gat gac ctc tcc cca gca gac acc gcg gca cta gac aca gat ctc 643
Ala Asp Asp Leu Ser Pro Ala Asp Thr Ala Ala Leu Asp Thr Asp Leu
                170                 175                 180
ttt gtg gga ctt gtc act gag ctg ggt ggc cca acg agc cac acc gcg 691
Phe Val Gly Leu Val Thr Glu Leu Gly Gly Pro Thr Ser His Thr Ala
            185                 190                 195

atc atc gca cgc cag ctc aac gtg cct tgc atc gtc gca tcc ggc gcc 739
Ile Ile Ala Arg Gln Leu Asn Val Pro Cys Ile Val Ala Ser Gly Ala
        200                 205                 210
ggc atc aag gac atc aag tcc ggc gaa aag gtg ctt atc gac ggc agc 787
Gly Ile Lys Asp Ile Lys Ser Gly Glu Lys Val Leu Ile Asp Gly Ser
    215                 220                 225
ctc ggc acc att gac cgc aac gcg gac gaa gct gaa gca acc aag ctc 835
Leu Gly Thr Ile Asp Arg Asn Ala Asp Glu Ala Glu Ala Thr Lys Leu
230                 235                 240                 245
gtc tcc gag tcc ctc gag cgc gct gct cgc atc gcc gag tgg aag ggt 883
Val Ser Glu Ser Leu Glu Arg Ala Ala Arg Ile Ala Glu Trp Lys Gly
                250                 255                 260
cct gca caa acc aag gac ggc tac cgc gtt cag ctg ttg gcc aac gtc 931
Pro Ala Gln Thr Lys Asp Gly Tyr Arg Val Gln Leu Leu Ala Asn Val
            265                 270                 275
caa gac ggc aac tct gca cag cag gct gca cag acc gaa gca gaa ggc 979
Gln Asp Gly Asn Ser Ala Gln Gln Ala Ala Gln Thr Glu Ala Glu Gly
        280                 285                 290
atc ggc ctg ttc cgc acc gaa ctg tgc ttc ctt tcc gcc acc gaa gag 1027
Ile Gly Leu Phe Arg Thr Glu Leu Cys Phe Leu Ser Ala Thr Glu Glu
    295                 300                 305
cca agc gtt gat gag cag gct gcg gtc tac tca aag gtg ctt gaa gca 1075
Pro Ser Val Asp Glu Gln Ala Ala Val Tyr Ser Lys Val Leu Glu Ala
310                 315                 320                 325
ttc cca gag tcc aag gtc gtt gtc cgc tcc ctc gac gca ggt tct gac 1123
Phe Pro Glu Ser Lys Val Val Val Arg Ser Leu Asp Ala Gly Ser Asp
                330                 335                 340
aag cca gtt cca ttc gca tcg atg gct gat gag atg aac cca gca ctg 1171
Lys Pro Val Pro Phe Ala Ser Met Ala Asp Glu Met Asn Pro Ala Leu
            345                 350                 355
ggt gtt cgt ggc ctg cgt atc gca cgt gga cag gtt gat ctg ctg act 1219
Gly Val Arg Gly Leu Arg Ile Ala Arg Gly Gln Val Asp Leu Leu Thr
        360                 365                 370
cgc cag ctc gac gca att gcg aag gcc agc gaa gaa ctc ggc cgt ggc 1267
Arg Gln Leu Asp Ala Ile Ala Lys Ala Ser Glu Glu Leu Gly Arg Gly
    375                 380                 385
gac gac gcc cca acc tgg gtt atg gct cca atg gtg gct acc gct tat 1315
Asp Asp Ala Pro Thr Trp Val Met Ala Pro Met Val Ala Thr Ala Tyr
390                 395                 400                 405
gaa gca aag tgg ttt gct gac atg tgc cgt gag cgt ggc cta atc gcc 1363
Glu Ala Lys Trp Phe Ala Asp Met Cys Arg Glu Arg Gly Leu Ile Ala
                410                 415                 420
ggc gcc atg atc gaa gtt cca gca gca tcc ctg atg gca gac aag atc 1411
Gly Ala Met Ile Glu Val Pro Ala Ala Ser Leu Met Ala Asp Lys Ile
            425                 430                 435
atg cct cac ctg gac ttt gtt tcc atc ggt acc aac gac ctg acc cag 1459
Met Pro His Leu Asp Phe Val Ser Ile Gly Thr Asn Asp Leu Thr Gln
        440                 445                 450
tac acc atg gca gcg gac cgc atg tct cct gag ctt gcc tac ctg acc 1507
Tyr Thr Met Ala Ala Asp Arg Met Ser Pro Glu Leu Ala Tyr Leu Thr
    455                 460                 465
gat cct tgg cag cca gca gtc ctg cgc ctg atc aag cac acc tgt gac 1555
Asp Pro Trp Gln Pro Ala Val Leu Arg Leu Ile Lys His Thr Cys Asp
470                 475                 480                 485
gaa ggt gct cgc ttt aac acc ccg gtc ggt gtt tgt ggt gaa gca gca 1603
Glu Gly Ala Arg Phe Asn Thr Pro Val Gly Val Cys Gly Glu Ala Ala
                490                 495                 500
gca gac cca ctg ttg gca act gtc ctc acc ggt ctt ggc gtg aac tcc 1651
Ala Asp Pro Leu Leu Ala Thr Val Leu Thr Gly Leu Gly Val Asn Ser
            505                 510                 515
ctg tcc gca gca tcc act gct ctc gca gca gtc ggt gca aag ctg tca 1699
Leu Ser Ala Ala Ser Thr Ala Leu Ala Ala Val Gly Ala Lys Leu Ser
        520                 525                 530
gag gtc acc ctg gaa acc tgt aag aag gca gca gaa gca gca ctt gac 1747
Glu Val Thr Leu Glu Thr Cys Lys Lys Ala Ala Glu Ala Ala Leu Asp
    535                 540                 545
gct gaa ggt gca act gaa gca cgc gat gct gta cgc gca gtg atc gac 1795
Ala Glu Gly Ala Thr Glu Ala Arg Asp Ala Val Arg Ala Val Ile Asp

550                                     555                                     560                                     565
gca gca gtc taaaccactg ttgagctaaa aagcctcaaa                                                    1834
Ala Ala Val

<210> 2
<211> 568
<212> PRT
<213> Corynebacterium glutamicum
<400> 2
Val Ala Thr Val Ala Asp Val Asn Gln Asp Thr Val Leu Lys Gly Thr
1               5                   10                  15
Gly Val Val Gly Gly Val Arg Tyr Ala Ser Ala Val Trp Ile Thr Pro
            20                  25                  30
Arg Pro Glu Leu Pro Gln Ala Gly Glu Val Val Ala Glu Glu Asn Arg
        35                  40                  45
Glu Ala Glu Gln Glu Arg Phe Asp Ala Ala Ala Ala Thr Val Ser Ser
    50                  55                  60
Arg Leu Leu Glu Arg Ser Glu Ala Ala Glu Gly Pro Ala Ala Glu Val
65                  70                  75                  80
Leu Lys Ala Thr Ala Gly Met Val Asn Asp Arg Gly Trp Arg Lys Ala
                85                  90                  95
Val Ile Lys Gly Val Lys Gly Gly His Pro Ala Glu Tyr Ala Val Val
            100                 105                 110
Ala Ala Thr Thr Lys Phe Ile Ser Met Phe Glu Ala Ala Gly Gly Leu
        115                 120                 125
Ile Ala Glu Arg Thr Thr Asp Leu Arg Asp Ile Arg Asp Arg Val Ile
    130                 135                 140
Ala Glu Leu Arg Gly Asp Glu Glu Pro Gly Leu Pro Ala Val Ser Gly
145                 150                 155                 160
Gln Val Ile Leu Phe Ala Asp Asp Leu Ser Pro Ala Asp Thr Ala Ala
                165                 170                 175
Leu Asp Thr Asp Leu Phe Val Gly Leu Val Thr Glu Leu Gly Gly Pro
            180                 185                 190
Thr Ser His Thr Ala Ile Ile Ala Arg Gln Leu Asn Val Pro Cys Ile
        195                 200                 205
Val Ala Ser Gly Ala Gly Ile Lys Asp Ile Lys Ser Gly Glu Lys Val
    210                 215                 220
Leu Ile Asp Gly Ser Leu Gly Thr Ile Asp Arg Asn Ala Asp Glu Ala
225                 230                 235                 240
Glu Ala Thr Lys Leu Val Ser Glu Ser Leu Glu Arg Ala Ala Arg Ile
                245                 250                 255
Ala Glu Trp Lys Gly Pro Ala Gln Thr Lys Asp Gly Tyr Arg Val Gln
            260                 265                 270
Leu Leu Ala Asn Val Gln Asp Gly Asn Ser Ala Gln Gln Ala Ala Gln
        275                 280                 285
Thr Glu Ala Glu Gly Ile Gly Leu Phe Arg Thr Glu Leu Cys Phe Leu
    290                 295                 300
Ser Ala Thr Glu Glu Pro Ser Val Asp Glu Gln Ala Ala Val Tyr Ser
305                 310                 315                 320
Lys Val Leu Glu Ala Phe Pro Glu Ser Lys Val Val Val Arg Ser Leu
                325                 330                 335
Asp Ala Gly Ser Asp Lys Pro Val Pro Phe Ala Ser Met Ala Asp Glu
            340                 345                 350
Met Asn Pro Ala Leu Gly Val Arg Gly Leu Arg Ile Ala Arg Gly Gln
        355                 360                 365
Val Asp Leu Leu Thr Arg Gln Leu Asp Ala Ile Ala Lys Ala Ser Glu
    370                 375                 380
Glu Leu Gly Arg Gly Asp Asp Ala Pro Thr Trp Val Met Ala Pro Met
385                 390                 395                 400

Val Ala Thr Ala Tyr Glu Ala Lys Trp Phe Ala Asp Met Cys Arg Glu
                405                 410                 415
Arg Gly Leu Ile Ala Gly Ala Met Ile Glu Val Pro Ala Ala Ser Leu
            420                 425                 430
Met Ala Asp Lys Ile Met Pro His Leu Asp Phe Val Ser Ile Gly Thr
        435                 440                 445
Asn Asp Leu Thr Gln Tyr Thr Met Ala Ala Asp Arg Met Ser Pro Glu

450                 455                 460
Leu Ala Tyr Leu Thr Asp Pro Trp Gln Pro Ala Val Leu Arg Leu Ile
465                 470                 475                 480
Lys His Thr Cys Asp Glu Gly Ala Arg Phe Asn Thr Pro Val Gly Val
                485                 490                 495
Cys Gly Glu Ala Ala Ala Asp Pro Leu Leu Ala Thr Val Leu Thr Gly
            500                 505                 510
Leu Gly Val Asn Ser Leu Ser Ala Ala Ser Thr Ala Leu Ala Ala Val
        515                 520                 525
Gly Ala Lys Leu Ser Glu Val Thr Leu Glu Thr Cys Lys Lys Ala Ala
    530                 535                 540
Glu Ala Ala Leu Asp Ala Glu Gly Ala Thr Glu Ala Arg Asp Ala Val
545                 550                 555                 560
Arg Ala Val Ile Asp Ala Ala Val
                565
<210> 3
<211> 397
<212> DNA
<213> Corynebacterium glutamicum
<220>
<221> CDS
<222> (101)..(367)
<223> RXA01300
<400> 3
gatcgacatt aaatcccctc ccttgggggg tttaactaac aaatcgctgc gccctaatcc 60
gttcggatta acggcgtagc aacacgaaag gacactttcc atg gct tcc aag act    115
                                            Met Ala Ser Lys Thr
                                            1               5
gta acc gtc ggt tcc tcc gtt ggc ctg cac gca cgt cca gca tcc atc    163
Val Thr Val Gly Ser Ser Val Gly Leu His Ala Arg Pro Ala Ser Ile
                10                  15                  20
atc gct gaa gcg gct gct gag tac gac gac gaa atc ttg ctg acc ctg    211
Ile Ala Glu Ala Ala Ala Glu Tyr Asp Asp Glu Ile Leu Leu Thr Leu
            25                  30                  35
gtt ggc tcc gat gat gac gaa gag acc gac gcg tcc tct tcc ctc atg    259
Val Gly Ser Asp Asp Asp Glu Glu Thr Asp Ala Ser Ser Ser Leu Met
        40                  45                  50

atc atg gcg ctg ggc gca gag cac ggc aac gaa gtt acc gtc acc tcc    307
Ile Met Ala Leu Gly Ala Glu His Gly Asn Glu Val Thr Val Thr Ser
    55                  60                  65
gac aac gct gaa gct gtt gag aag atc gct gcg ctt atc gca cag gac    355
Asp Asn Ala Glu Ala Val Glu Lys Ile Ala Ala Leu Ile Ala Gln Asp
70                  75                  80                  85
ctt gac gct gag taaacaacgc tctgcttgtt aaaagctcgt                   397
Leu Asp Ala Glu

<210> 4
<211> 89
<212> PRT
<213> Corynebacterium glutamicum
<400> 4
Met Ala Ser Lys Thr Val Thr Val Gly Ser Ser Val Gly Leu His Ala
1               5                   10                  15
Arg Pro Ala Ser Ile Ile Ala Glu Ala Ala Ala Glu Tyr Asp Asp Glu
            20                  25                  30
Ile Leu Leu Thr Leu Val Gly Ser Asp Asp Asp Glu Glu Thr Asp Ala
        35                  40                  45
Ser Ser Ser Leu Met Ile Met Ala Leu Gly Ala Glu His Gly Asn Glu
    50                  55                  60
Val Thr Val Thr Ser Asp Asn Ala Glu Ala Val Glu Lys Ile Ala Ala
65                  70                  75                  80
Leu Ile Ala Gln Asp Leu Asp Ala Glu
                85
<210> 5
<211> 940
<212> DNA
<213> Corynebacterium glutamicum

<220>
<221> CDS
<222> (101)..(910)
<223> RXA04358
<400> 5
ggaacttcga ggtgtcttcg tggggcgtac ggagatctag caagtgtggc tttatgtttg 60
accctatccg aatcaacatg cagtgaatta acatctactt atg ttt gta ctc aaa 115
Met Phe Val Leu Lys
1               5
gat ctg cta aag gca gaa cgc ata gaa ctc gac cgc acg gtc acc gat 163
Asp Leu Leu Lys Ala Glu Arg Ile Glu Leu Asp Arg Thr Val Thr Asp
                10                  15                  20
tgg cgt gaa ggc atc cgc gcc gca ggt gta ctc cta gaa aag aca aac 211
Trp Arg Glu Gly Ile Arg Ala Ala Gly Val Leu Leu Glu Lys Thr Asn
            25                  30                  35
agc att gat tcc gcc tac acc gat gcc atg atc gcc agc gtg gaa gaa 259
Ser Ile Asp Ser Ala Tyr Thr Asp Ala Met Ile Ala Ser Val Glu Glu
        40                  45                  50
aaa ggc ccc tac att gtg gtc gct cca ggt ttc gct ttc gcg cac gcc 307
Lys Gly Pro Tyr Ile Val Val Ala Pro Gly Phe Ala Phe Ala His Ala
    55                  60                  65
cgc ccc agc aga gca gtc cgc gag acc gct atg tcg tgg gtg cgc ctg 355
Arg Pro Ser Arg Ala Val Arg Glu Thr Ala Met Ser Trp Val Arg Leu
70                  75                  80                  85
gcc tcc cct gtt tcc ttc ggt cac agt aag aat gat ccc gtc aat ctc 403
Ala Ser Pro Val Ser Phe Gly His Ser Lys Asn Asp Pro Val Asn Leu
                90                  95                  100
atc gtt gct ctc gct gcc aaa gat gcc acc gca cat acc caa gcg atg 451
Ile Val Ala Leu Ala Ala Lys Asp Ala Thr Ala His Thr Gln Ala Met
            105                 110                 115
gcg gca ttg gct aaa gct tta gga aaa tac cga aag gat ctc gac gag 499
Ala Ala Leu Ala Lys Ala Leu Gly Lys Tyr Arg Lys Asp Leu Asp Glu
        120                 125                 130
gca caa agt ccc gag gag atc caa gca atc tta gag aag gca gca gcg 547
Ala Gln Ser Pro Glu Glu Ile Gln Ala Ile Leu Glu Lys Ala Ala Ala
    135                 140                 145
ccg gcg aag cag aag gct cct gct gtg gct cct gct gta aca ccc act 595
Pro Ala Lys Gln Lys Ala Pro Ala Val Ala Pro Ala Val Thr Pro Thr
150                 155                 160                 165
gac gct cct gca gcc tca gtc caa tcc aaa acc cac gac aag atc ctc 643
Asp Ala Pro Ala Ala Ser Val Gln Ser Lys Thr His Asp Lys Ile Leu
                170                 175                 180
acc gtc tgt ggc aac ggc ttg ggt acc tcc ctc ttc ctc aaa aac acc 691
Thr Val Cys Gly Asn Gly Leu Gly Thr Ser Leu Phe Leu Lys Asn Thr
            185                 190                 195
ctt gag caa gtt ttc gac acc tgg ggt tgg ggt cca tac atg acg gtg 739
Leu Glu Gln Val Phe Asp Thr Trp Gly Trp Gly Pro Tyr Met Thr Val
        200                 205                 210
gag gca acc gac act atc tcc gcc aag ggc aaa gcc aag gaa gct gat 787
Glu Ala Thr Asp Thr Ile Ser Ala Lys Gly Lys Ala Lys Glu Ala Asp
    215                 220                 225
ctc atc atg acc tct ggt gaa atc gcc cgc acg ttg ggt gat gtt gga 835
Leu Ile Met Thr Ser Gly Glu Ile Ala Arg Thr Leu Gly Asp Val Gly
230                 235                 240                 245
atc ccg gtt cac gtg atc aat gac ttc acg agc acc gat gaa atc gat 883
Ile Pro Val His Val Ile Asn Asp Phe Thr Ser Thr Asp Glu Ile Asp
                250                 255                 260
gct gcg ctt cgt gaa cgc tac gac atc taactacttt aaaaggacga 930
Ala Ala Leu Arg Glu Arg Tyr Asp Ile
            265                 270
aaatattatg 940

<210> 6
<211> 270
<212> PRT
<213> Corynebacterium glutamicum
<400> 6

Met Phe Val Leu Lys Asp Leu Leu Lys Ala Glu Arg Ile Glu Leu Asp
1               5                   10                  15
Arg Thr Val Thr Asp Trp Arg Glu Gly Ile Arg Ala Ala Gly Val Leu
            20                  25                  30
Leu Glu Lys Thr Asn Ser Ile Asp Ser Ala Tyr Thr Asp Ala Met Ile
        35                  40                  45
Ala Ser Val Glu Glu Lys Gly Pro Tyr Ile Val Val Ala Pro Gly Phe
    50                  55                  60
Ala Phe Ala His Ala Arg Pro Ser Arg Ala Val Arg Glu Thr Ala Met
65                  70                  75                  80
Ser Trp Val Arg Leu Ala Ser Pro Val Ser Phe Gly His Ser Lys Asn
                85                  90                  95
Asp Pro Val Asn Leu Ile Val Ala Leu Ala Ala Lys Asp Ala Thr Ala
            100                 105                 110
His Thr Gln Ala Met Ala Ala Leu Ala Lys Ala Leu Gly Lys Tyr Arg
        115                 120                 125
Lys Asp Leu Asp Glu Ala Gln Ser Pro Glu Glu Ile Gln Ala Ile Leu
    130                 135                 140
Glu Lys Ala Ala Ala Pro Ala Lys Gln Lys Ala Pro Ala Val Ala Pro
145                 150                 155                 160
Ala Val Thr Pro Thr Asp Ala Pro Ala Ala Ser Val Gln Ser Lys Thr
                165                 170                 175
His Asp Lys Ile Leu Thr Val Cys Gly Asn Gly Leu Gly Thr Ser Leu
            180                 185                 190
Phe Leu Lys Asn Thr Leu Glu Gln Val Phe Asp Thr Trp Gly Trp Gly
        195                 200                 205
Pro Tyr Met Thr Val Glu Ala Thr Asp Thr Ile Ser Ala Lys Gly Lys
    210                 215                 220
Ala Lys Glu Ala Asp Leu Ile Met Thr Ser Gly Glu Ile Ala Arg Thr
225                 230                 235                 240
Leu Gly Asp Val Gly Ile Pro Val His Val Ile Asn Asp Phe Thr Ser
                245                 250                 255
Thr Asp Glu Ile Asp Ala Ala Leu Arg Glu Arg Tyr Asp Ile
            260                 265                 270
<210> 7
<211> 520
<212> DNA
<213> Corynebacterium glutamicum

<220>
<221> CDS
<222> (101)..(490)
<223> RXA06018
<400> 7
agcctacctc tttggtaccg caggcctgtc taccggcgac caagcttcca tggaaatcat 60
ggccgcgatc atggcagctg gcatggtccc accaatcgcg ttg tcc att gct acc    115
                                            Leu Ser Ile Ala Thr
                                            1               5
ctg ctg cgc aag aag ctg ttc acc cca gca gag caa gaa aac ggc aag    163
Leu Leu Arg Lys Lys Leu Phe Thr Pro Ala Glu Gln Glu Asn Gly Lys
                10                  15                  20
tct tcc tgg ctg ctt ggc ctg gca ttc gtc tcc gaa ggt gcc atc cca    211
Ser Ser Trp Leu Leu Gly Leu Ala Phe Val Ser Glu Gly Ala Ile Pro
            25                  30                  35
ttc gcc gca gct gac cca ttc cgt gtg atc cca gca atg atg gct ggc    259
Phe Ala Ala Ala Asp Pro Phe Arg Val Ile Pro Ala Met Met Ala Gly
        40                  45                  50
ggt gca acc act ggt gca atc tcc atg gca ctg ggc gtc ggc tct cgg    307
Gly Ala Thr Thr Gly Ala Ile Ser Met Ala Leu Gly Val Gly Ser Arg
    55                  60                  65
gct cca cac ggc ggt atc ttc gtg gtc tgg gca atc gaa cca tgg tgg    355
Ala Pro His Gly Gly Ile Phe Val Val Trp Ala Ile Glu Pro Trp Trp
70                  75                  80                  85
ggc tgg ctc atc gca ctt gca gca ggc acc atc gtg tcc acc atc gtt    403
Gly Trp Leu Ile Ala Leu Ala Ala Gly Thr Ile Val Ser Thr Ile Val
                90                  95                  100
gtc atc gca ctg aag cag ttc tgg cca aac aag gcc gtc gct gca gaa    451
Val Ile Ala Leu Lys Gln Phe Trp Pro Asn Lys Ala Val Ala Ala Glu

            105                 110                 115
gtc gcg aag caa aaa gca caa caa gca gct gta aac gca taatcggacc 500
Val Ala Lys Gln Lys Ala Gln Gln Ala Ala Val Asn Ala
        120                 125                 130
ttgacccgat gtctggtcgg 520
<210> 8
<211> 130
<212> PRT
<213> Corynebacterium glutamicum
<400> 8
Leu Ser Ile Ala Thr Leu Leu Arg Lys Lys Leu Phe Thr Pro Ala Glu
1               5                   10                  15
Gln Glu Asn Gly Lys Ser Ser Trp Leu Leu Gly Leu Ala Phe Val Ser
            20                  25                  30
Glu Gly Ala Ile Pro Phe Ala Ala Ala Asp Pro Phe Arg Val Ile Pro
        35                  40                  45

Ala Met Met Ala Gly Gly Ala Thr Thr Gly Ala Ile Ser Met Ala Leu
    50                  55                  60
Gly Val Gly Ser Arg Ala Pro His Gly Gly Ile Phe Val Val Trp Ala
65                  70                  75                  80
Ile Glu Pro Trp Trp Gly Trp Leu Ile Ala Leu Ala Ala Gly Thr Ile
                85                  90                  95
Val Ser Thr Ile Val Val Ile Ala Leu Lys Gln Phe Trp Pro Asn Lys
            100                 105                 110
Ala Val Ala Ala Glu Val Ala Lys Gln Lys Ala Gln Gln Ala Ala Val
        115                 120                 125
Asn Ala
    130

SEQUENCE LISTING

<110> BASF Aktiengesellschaft

<120> Gene die für Phosphoenolpyruvat-Zucker-
      phosphotransferase Proteine codieren

<130> O.Z.0050/52969

<160> 8

<210> 1
<211> 1834
<212> DNA
<213> Corynebacterium glutamicum

<220>
<221> CDS
<222> (101)..(1804)
<223> RXA01244

<400> 1
gatatgtgtt tgtttgtcaa tatccaaatg tttgaatagt tgcacaactg ttggttttgt 60

ggtgatcttg aggaaattaa ctcaatgatt gtgaggatgg gtg gct act gtg gct   115
                                            Val Ala Thr Val Ala
                                            1               5

gat gtg aat caa gac act gta ctg aag ggc acc ggc gtt gtc ggt gga   163
Asp Val Asn Gln Asp Thr Val Leu Lys Gly Thr Gly Val Val Gly Gly
                10                  15                  20

gtc cgt tat gca agc gcg gtg tgg att acc cca cgc ccc gaa cta ccc   211
Val Arg Tyr Ala Ser Ala Val Trp Ile Thr Pro Arg Pro Glu Leu Pro
            25                  30                  35

caa gca ggc gaa gtc gtc gcc gaa gaa aac cgt gaa gca gag cag gag   259
Gln Ala Gly Glu Val Val Ala Glu Glu Asn Arg Glu Ala Glu Gln Glu
        40                  45                  50

cgt ttc gac gcc gct gca gcc aca gtc tct tct cgt ttg ctt gag cgc   307
Arg Phe Asp Ala Ala Ala Ala Thr Val Ser Ser Arg Leu Leu Glu Arg
    55                  60                  65

tcc gaa gct gct gaa gga cca gca gct gag gtg ctt aaa gct act gct   355
Ser Glu Ala Ala Glu Gly Pro Ala Ala Glu Val Leu Lys Ala Thr Ala
70                  75                  80                  85

ggc atg gtc aat gac cgt ggc tgg cgt aag gct gtc atc aag ggt gtc   403
Gly Met Val Asn Asp Arg Gly Trp Arg Lys Ala Val Ile Lys Gly Val
                90                  95                  100

aag ggt ggt cac cct gcg gaa tac gcc gtg gtt gca gca aca acc aag   451
Lys Gly Gly His Pro Ala Glu Tyr Ala Val Val Ala Ala Thr Thr Lys
            105                 110                 115

ttc atc tcc atg ttc gaa gcc gca ggc ggc ctg atc gcg gag cgc acc   499
Phe Ile Ser Met Phe Glu Ala Ala Gly Gly Leu Ile Ala Glu Arg Thr
        120                 125                 130

aca gac ttg cgc gac atc cgc gac cgc gtc atc gca gaa ctt cgt ggc   547
Thr Asp Leu Arg Asp Ile Arg Asp Arg Val Ile Ala Glu Leu Arg Gly
    135                 140                 145

gat gaa gag cca ggt ctg cca gct gtt tcc gga cag gtc att ctc ttt     595
Asp Glu Glu Pro Gly Leu Pro Ala Val Ser Gly Gln Val Ile Leu Phe
150                 155                 160                 165

gca gat gac ctc tcc cca gca gac acc gcg gca cta gac aca gat ctc     643
Ala Asp Asp Leu Ser Pro Ala Asp Thr Ala Ala Leu Asp Thr Asp Leu
                170                 175                 180

ttt gtg gga ctt gtc act gag ctg ggt ggc cca acg agc cac acc gcg     691
Phe Val Gly Leu Val Thr Glu Leu Gly Gly Pro Thr Ser His Thr Ala
            185                 190                 195

atc atc gca cgc cag ctc aac gtg cct tgc atc gtc gca tcc ggc gcc     739
Ile Ile Ala Arg Gln Leu Asn Val Pro Cys Ile Val Ala Ser Gly Ala
        200                 205                 210

ggc atc aag gac atc aag tcc ggc gaa aag gtg ctt atc gac ggc agc     787
Gly Ile Lys Asp Ile Lys Ser Gly Glu Lys Val Leu Ile Asp Gly Ser
    215                 220                 225

ctc ggc acc att gac cgc aac gcg gac gaa gct gaa gca acc aag ctc     835
Leu Gly Thr Ile Asp Arg Asn Ala Asp Glu Ala Glu Ala Thr Lys Leu
230                 235                 240                 245

gtc tcc gag tcc ctc gag cgc gct gct cgc atc gcc gag tgg aag ggt     883
Val Ser Glu Ser Leu Glu Arg Ala Ala Arg Ile Ala Glu Trp Lys Gly
                250                 255                 260

cct gca caa acc aag gac ggc tac cgc gtt cag ctg ttg gcc aac gtc     931
Pro Ala Gln Thr Lys Asp Gly Tyr Arg Val Gln Leu Leu Ala Asn Val
            265                 270                 275

caa gac ggc aac tct gca cag cag gct gca cag acc gaa gca gaa ggc     979
Gln Asp Gly Asn Ser Ala Gln Gln Ala Ala Gln Thr Glu Ala Glu Gly
        280                 285                 290

atc ggc ctg ttc cgc acc gaa ctg tgc ttc ctt tcc gcc acc gaa gag     1027
Ile Gly Leu Phe Arg Thr Glu Leu Cys Phe Leu Ser Ala Thr Glu Glu
    295                 300                 305

cca agc gtt gat gag cag gct gcg gtc tac tca aag gtg ctt gaa gca     1075
Pro Ser Val Asp Glu Gln Ala Ala Val Tyr Ser Lys Val Leu Glu Ala
310                 315                 320                 325

ttc cca gag tcc aag gtc gtt gtc cgc tcc ctc gac gca ggt tct gac     1123
Phe Pro Glu Ser Lys Val Val Val Arg Ser Leu Asp Ala Gly Ser Asp
                330                 335                 340

aag cca gtt cca ttc gca tcg atg gct gat gag atg aac cca gca ctg     1171
Lys Pro Val Pro Phe Ala Ser Met Ala Asp Glu Met Asn Pro Ala Leu
            345                 350                 355

ggt gtt cgt ggc ctg cgt atc gca cgt gga cag gtt gat ctg ctg act     1219
Gly Val Arg Gly Leu Arg Ile Ala Arg Gly Gln Val Asp Leu Leu Thr
        360                 365                 370

cgc cag ctc gac gca att gcg aag gcc agc gaa gaa ctc ggc cgt ggc     1267
Arg Gln Leu Asp Ala Ile Ala Lys Ala Ser Glu Glu Leu Gly Arg Gly
    375                 380                 385

gac gac gcc cca acc tgg gtt atg gct cca atg gtg gct acc gct tat     1315
Asp Asp Ala Pro Thr Trp Val Met Ala Pro Met Val Ala Thr Ala Tyr
390                 395                 400                 405

gaa gca aag tgg ttt gct gac atg tgc cgt gag cgt ggc cta atc gcc     1363
Glu Ala Lys Trp Phe Ala Asp Met Cys Arg Glu Arg Gly Leu Ile Ala
                410                 415                 420

ggc gcc atg atc gaa gtt cca gca gca tcc ctg atg gca gac aag atc     1411
Gly Ala Met Ile Glu Val Pro Ala Ala Ser Leu Met Ala Asp Lys Ile
            425                 430                 435

atg cct cac ctg gac ttt gtt tcc atc ggt acc aac gac ctg acc cag     1459
Met Pro His Leu Asp Phe Val Ser Ile Gly Thr Asn Asp Leu Thr Gln
        440                 445                 450

tac acc atg gca gcg gac cgc atg tct cct gag ctt gcc tac ctg acc     1507
Tyr Thr Met Ala Ala Asp Arg Met Ser Pro Glu Leu Ala Tyr Leu Thr
    455                 460                 465

gat cct tgg cag cca gca gtc ctg cgc ctg atc aag cac acc tgt gac     1555
Asp Pro Trp Gln Pro Ala Val Leu Arg Leu Ile Lys His Thr Cys Asp
470                 475                 480                 485

gaa ggt gct cgc ttt aac acc ccg gtc ggt gtt tgt ggt gaa gca gca     1603
Glu Gly Ala Arg Phe Asn Thr Pro Val Gly Val Cys Gly Glu Ala Ala
                490                 495                 500

gca gac cca ctg ttg gca act gtc ctc acc ggt ctt ggc gtg aac tcc     1651
Ala Asp Pro Leu Leu Ala Thr Val Leu Thr Gly Leu Gly Val Asn Ser
            505                 510                 515

ctg tcc gca gca tcc act gct ctc gca gca gtc ggt gca aag ctg tca     1699
Leu Ser Ala Ala Ser Thr Ala Leu Ala Ala Val Gly Ala Lys Leu Ser
        520                 525                 530

gag gtc acc ctg gaa acc tgt aag aag gca gca gaa gca gca ctt gac     1747
Glu Val Thr Leu Glu Thr Cys Lys Lys Ala Ala Glu Ala Ala Leu Asp
    535                 540                 545

gct gaa ggt gca act gaa gca cgc gat gct gta cgc gca gtg atc gac     1795
Ala Glu Gly Ala Thr Glu Ala Arg Asp Ala Val Arg Ala Val Ile Asp
550                 555                 560                 565

gca gca gtc taaaccactg ttgagctaaa aagcctcaaa                        1834
Ala Ala Val

<210> 2
<211> 568
<212> PRT
<213> Corynebacterium glutamicum

<400> 2
Val Ala Thr Val Ala Asp Val Asn Gln Asp Thr Val Leu Lys Gly Thr
1               5                   10                  15

Gly Val Val Gly Gly Val Arg Tyr Ala Ser Ala Val Trp Ile Thr Pro
            20                  25                  30

Arg Pro Glu Leu Pro Gln Ala Gly Glu Val Val Ala Glu Glu Asn Arg
        35                  40                  45

Glu Ala Glu Gln Glu Arg Phe Asp Ala Ala Ala Ala Thr Val Ser Ser
    50                  55                  60

Arg Leu Leu Glu Arg Ser Glu Ala Ala Glu Gly Pro Ala Ala Glu Val
65                  70                  75                  80

Leu Lys Ala Thr Ala Gly Met Val Asn Asp Arg Gly Trp Arg Lys Ala
                85                  90                  95

Val Ile Lys Gly Val Lys Gly Gly His Pro Ala Glu Tyr Ala Val Val
            100                 105                 110

Ala Ala Thr Thr Lys Phe Ile Ser Met Phe Glu Ala Ala Gly Gly Leu
        115                 120                 125

Ile Ala Glu Arg Thr Thr Asp Leu Arg Asp Ile Arg Asp Arg Val Ile
    130                 135                 140

Ala Glu Leu Arg Gly Asp Glu Glu Pro Gly Leu Pro Ala Val Ser Gly
145                 150                 155                 160

Gln Val Ile Leu Phe Ala Asp Asp Leu Ser Pro Ala Asp Thr Ala Ala
                165                 170                 175

Leu Asp Thr Asp Leu Phe Val Gly Leu Val Thr Glu Leu Gly Gly Pro
            180                 185                 190

Thr Ser His Thr Ala Ile Ile Ala Arg Gln Leu Asn Val Pro Cys Ile
        195                 200                 205

Val Ala Ser Gly Ala Gly Ile Lys Asp Ile Lys Ser Gly Glu Lys Val
    210                 215                 220

Leu Ile Asp Gly Ser Leu Gly Thr Ile Asp Arg Asn Ala Asp Glu Ala
225                 230                 235                 240

Glu Ala Thr Lys Leu Val Ser Glu Ser Leu Glu Arg Ala Ala Arg Ile
                245                 250                 255

Ala Glu Trp Lys Gly Pro Ala Gln Thr Lys Asp Gly Tyr Arg Val Gln
            260                 265                 270

Leu Leu Ala Asn Val Gln Asp Gly Asn Ser Ala Gln Gln Ala Ala Gln
        275                 280                 285

Thr Glu Ala Glu Gly Ile Gly Leu Phe Arg Thr Glu Leu Cys Phe Leu
    290                 295                 300

Ser Ala Thr Glu Glu Pro Ser Val Asp Glu Gln Ala Ala Val Tyr Ser
305                 310                 315                 320

Lys Val Leu Glu Ala Phe Pro Glu Ser Lys Val Val Val Arg Ser Leu
                325                 330                 335

Asp Ala Gly Ser Asp Lys Pro Val Pro Phe Ala Ser Met Ala Asp Glu
            340                 345                 350

Met Asn Pro Ala Leu Gly Val Arg Gly Leu Arg Ile Ala Arg Gly Gln
        355                 360                 365

Val Asp Leu Leu Thr Arg Gln Leu Asp Ala Ile Ala Lys Ala Ser Glu
    370                 375                 380

Glu Leu Gly Arg Gly Asp Asp Ala Pro Thr Trp Val Met Ala Pro Met
385                 390                 395                 400

Val Ala Thr Ala Tyr Glu Ala Lys Trp Phe Ala Asp Met Cys Arg Glu

                405                 410                 415

Arg Gly Leu Ile Ala Gly Ala Met Ile Glu Val Pro Ala Ala Ser Leu
            420                 425                 430

Met Ala Asp Lys Ile Met Pro His Leu Asp Phe Val Ser Ile Gly Thr
        435                 440                 445

Asn Asp Leu Thr Gln Tyr Thr Met Ala Ala Asp Arg Met Ser Pro Glu
    450                 455                 460

Leu Ala Tyr Leu Thr Asp Pro Trp Gln Pro Ala Val Leu Arg Leu Ile
465                 470                 475                 480

Lys His Thr Cys Asp Glu Gly Ala Arg Phe Asn Thr Pro Val Gly Val
                485                 490                 495

Cys Gly Glu Ala Ala Ala Asp Pro Leu Leu Ala Thr Val Leu Thr Gly
            500                 505                 510

Leu Gly Val Asn Ser Leu Ser Ala Ala Ser Thr Ala Leu Ala Ala Val
        515                 520                 525

Gly Ala Lys Leu Ser Glu Val Thr Leu Glu Thr Cys Lys Lys Ala Ala
    530                 535                 540

Glu Ala Ala Leu Asp Ala Glu Gly Ala Thr Glu Ala Arg Asp Ala Val
545                 550                 555                 560

Arg Ala Val Ile Asp Ala Ala Val
                565

<210> 3
<211> 397
<212> DNA
<213> Corynebacterium glutamicum

<220>
<221> CDS
<222> (101)..(367)
<223> RXA01300

<400> 3
gatcgacatt aaatcccctc ccttgggggg tttaactaac aaatcgctgc gccctaatcc 60

gttcggatta acggcgtagc aacacgaaag gacactttcc atg gct tcc aag act 115
Met Ala Ser Lys Thr
1               5

gta acc gtc ggt tcc tcc gtt ggc ctg cac gca cgt cca gca tcc atc 163
Val Thr Val Gly Ser Ser Val Gly Leu His Ala Arg Pro Ala Ser Ile
                10                  15                  20

atc gct gaa gcg gct gct gag tac gac gac gaa atc ttg ctg acc ctg 211
Ile Ala Glu Ala Ala Ala Glu Tyr Asp Asp Glu Ile Leu Leu Thr Leu
            25                  30                  35

gtt ggc tcc gat gat gac gaa gag acc gac gcg tcc tct tcc ctc atg 259
Val Gly Ser Asp Asp Asp Glu Glu Thr Asp Ala Ser Ser Ser Leu Met
        40                  45                  50

atc atg gcg ctg ggc gca gag cac ggc aac gaa gtt acc gtc acc tcc 307

Ile Met Ala Leu Gly Ala Glu His Gly Asn Glu Val Thr Val Thr Ser
     55                  60                  65

gac aac gct gaa gct gtt gag aag atc gct gcg ctt atc gca cag gac     355
Asp Asn Ala Glu Ala Val Glu Lys Ile Ala Ala Leu Ile Ala Gln Asp
 70                  75                  80                  85

ctt gac gct gag taaacaacgc tctgcttgtt aaaagctcgt                    397
Leu Asp Ala Glu

<210> 4
<211> 89
<212> PRT
<213> Corynebacterium glutamicum

<400> 4
Met Ala Ser Lys Thr Val Thr Val Gly Ser Ser Val Gly Leu His Ala
  1               5                  10                  15

Arg Pro Ala Ser Ile Ile Ala Glu Ala Ala Ala Glu Tyr Asp Asp Glu
             20                  25                  30

Ile Leu Leu Thr Leu Val Gly Ser Asp Asp Asp Glu Glu Thr Asp Ala
         35                  40                  45

Ser Ser Ser Leu Met Ile Met Ala Leu Gly Ala Glu His Gly Asn Glu
     50                  55                  60

Val Thr Val Thr Ser Asp Asn Ala Glu Ala Val Glu Lys Ile Ala Ala
 65                  70                  75                  80

Leu Ile Ala Gln Asp Leu Asp Ala Glu
                 85

<210> 5
<211> 940
<212> DNA
<213> Corynebacterium glutamicum

<220>
<221> CDS
<222> (101)..(910)
<223> RXA04358

<400> 5
ggaacttcga ggtgtcttcg tggggcgtac ggagatctag caagtgtggc tttatgtttg 60

accctatccg aatcaacatg cagtgaatta acatctactt atg ttt gta ctc aaa     115
                                           Met Phe Val Leu Lys
                                             1               5

gat ctg cta aag gca gaa cgc ata gaa ctc gac cgc acg gtc acc gat     163
Asp Leu Leu Lys Ala Glu Arg Ile Glu Leu Asp Arg Thr Val Thr Asp
                 10                  15                  20

tgg cgt gaa ggc atc cgc gcc gca ggt gta ctc cta gaa aag aca aac     211
Trp Arg Glu Gly Ile Arg Ala Ala Gly Val Leu Leu Glu Lys Thr Asn
             25                  30                  35

agc att gat tcc gcc tac acc gat gcc atg atc gcc agc gtg gaa gaa     259

Ser Ile Asp Ser Ala Tyr Thr Asp Ala Met Ile Ala Ser Val Glu Glu
        40                  45                  50

aaa ggc ccc tac att gtg gtc gct cca ggt ttc gct ttc gcg cac gcc     307
Lys Gly Pro Tyr Ile Val Val Ala Pro Gly Phe Ala Phe Ala His Ala
    55                  60                  65

cgc ccc agc aga gca gtc cgc gag acc gct atg tcg tgg gtg cgc ctg     355
Arg Pro Ser Arg Ala Val Arg Glu Thr Ala Met Ser Trp Val Arg Leu
70                  75                  80                  85

gcc tcc cct gtt tcc ttc ggt cac agt aag aat gat ccc gtc aat ctc     403
Ala Ser Pro Val Ser Phe Gly His Ser Lys Asn Asp Pro Val Asn Leu
                90                  95                  100

atc gtt gct ctc gct gcc aaa gat gcc acc gca cat acc caa gcg atg     451
Ile Val Ala Leu Ala Ala Lys Asp Ala Thr Ala His Thr Gln Ala Met
            105                 110                 115

gcg gca ttg gct aaa gct tta gga aaa tac cga aag gat ctc gac gag     499
Ala Ala Leu Ala Lys Ala Leu Gly Lys Tyr Arg Lys Asp Leu Asp Glu
        120                 125                 130

gca caa agt ccc gag gag atc caa gca atc tta gag aag gca gca gcg     547
Ala Gln Ser Pro Glu Glu Ile Gln Ala Ile Leu Glu Lys Ala Ala Ala
    135                 140                 145

ccg gcg aag cag aag gct cct gct gtg gct cct gct gta aca ccc act     595
Pro Ala Lys Gln Lys Ala Pro Ala Val Ala Pro Ala Val Thr Pro Thr
150                 155                 160                 165

gac gct cct gca gcc tca gtc caa tcc aaa acc cac gac aag atc ctc     643
Asp Ala Pro Ala Ala Ser Val Gln Ser Lys Thr His Asp Lys Ile Leu
                170                 175                 180

acc gtc tgt ggc aac ggc ttg ggt acc tcc ctc ttc ctc aaa aac acc     691
Thr Val Cys Gly Asn Gly Leu Gly Thr Ser Leu Phe Leu Lys Asn Thr
            185                 190                 195

ctt gag caa gtt ttc gac acc tgg ggt tgg ggt cca tac atg acg gtg     739
Leu Glu Gln Val Phe Asp Thr Trp Gly Trp Gly Pro Tyr Met Thr Val
        200                 205                 210

gag gca acc gac act atc tcc gcc aag ggc aaa gcc aag gaa gct gat     787
Glu Ala Thr Asp Thr Ile Ser Ala Lys Gly Lys Ala Lys Glu Ala Asp
    215                 220                 225

ctc atc atg acc tct ggt gaa atc gcc cgc acg ttg ggt gat gtt gga     835
Leu Ile Met Thr Ser Gly Glu Ile Ala Arg Thr Leu Gly Asp Val Gly
230                 235                 240                 245

atc ccg gtt cac gtg atc aat gac ttc acg agc acc gat gaa atc gat     883
Ile Pro Val His Val Ile Asn Asp Phe Thr Ser Thr Asp Glu Ile Asp
                250                 255                 260

gct gcg ctt cgt gaa cgc tac gac atc taactacttt aaaaggacga           930
Ala Ala Leu Arg Glu Arg Tyr Asp Ile
            265                 270

aaatattatg                                                          940

<210> 6

<211> 270
<212> PRT
<213> Corynebacterium glutamicum

<400> 6
Met Phe Val Leu Lys Asp Leu Leu Lys Ala Glu Arg Ile Glu Leu Asp
1               5                   10                  15

Arg Thr Val Thr Asp Trp Arg Glu Gly Ile Arg Ala Ala Gly Val Leu
            20                  25                  30

Leu Glu Lys Thr Asn Ser Ile Asp Ser Ala Tyr Thr Asp Ala Met Ile
        35                  40                  45

Ala Ser Val Glu Glu Lys Gly Pro Tyr Ile Val Val Ala Pro Gly Phe
    50                  55                  60

Ala Phe Ala His Ala Arg Pro Ser Arg Ala Val Arg Glu Thr Ala Met
65                  70                  75                  80

Ser Trp Val Arg Leu Ala Ser Pro Val Ser Phe Gly His Ser Lys Asn
                85                  90                  95

Asp Pro Val Asn Leu Ile Val Ala Leu Ala Ala Lys Asp Ala Thr Ala
            100                 105                 110

His Thr Gln Ala Met Ala Ala Leu Ala Lys Ala Leu Gly Lys Tyr Arg
        115                 120                 125

Lys Asp Leu Asp Glu Ala Gln Ser Pro Glu Glu Ile Gln Ala Ile Leu
    130                 135                 140

Glu Lys Ala Ala Ala Pro Ala Lys Gln Lys Ala Pro Ala Val Ala Pro
145                 150                 155                 160

Ala Val Thr Pro Thr Asp Ala Pro Ala Ala Ser Val Gln Ser Lys Thr
                165                 170                 175

His Asp Lys Ile Leu Thr Val Cys Gly Asn Gly Leu Gly Thr Ser Leu
            180                 185                 190

Phe Leu Lys Asn Thr Leu Glu Gln Val Phe Asp Thr Trp Gly Trp Gly
        195                 200                 205

Pro Tyr Met Thr Val Glu Ala Thr Asp Thr Ile Ser Ala Lys Gly Lys
    210                 215                 220

Ala Lys Glu Ala Asp Leu Ile Met Thr Ser Gly Glu Ile Ala Arg Thr
225                 230                 235                 240

Leu Gly Asp Val Gly Ile Pro Val His Val Ile Asn Asp Phe Thr Ser
                245                 250                 255

Thr Asp Glu Ile Asp Ala Ala Leu Arg Glu Arg Tyr Asp Ile
            260                 265                 270

<210> 7
<211> 520
<212> DNA
<213> Corynebacterium glutamicum

<220>

<221> CDS
<222> (101)..(490)
<223> RXA06018

<400> 7
agcctacctc tttggtaccg caggcctgtc taccggcgac caagcttcca tggaaatcat 60

ggccgcgatc atggcagctg gcatggtccc accaatcgcg ttg tcc att gct acc 115
Leu Ser Ile Ala Thr
1               5

ctg ctg cgc aag aag ctg ttc acc cca gca gag caa gaa aac ggc aag 163
Leu Leu Arg Lys Lys Leu Phe Thr Pro Ala Glu Gln Glu Asn Gly Lys
                10                  15                  20

tct tcc tgg ctg ctt ggc ctg gca ttc gtc tcc gaa ggt gcc atc cca 211
Ser Ser Trp Leu Leu Gly Leu Ala Phe Val Ser Glu Gly Ala Ile Pro
            25                  30                  35

ttc gcc gca gct gac cca ttc cgt gtg atc cca gca atg atg gct ggc 259
Phe Ala Ala Ala Asp Pro Phe Arg Val Ile Pro Ala Met Met Ala Gly
        40                  45                  50

ggt gca acc act ggt gca atc tcc atg gca ctg ggc gtc ggc tct cgg 307
Gly Ala Thr Thr Gly Ala Ile Ser Met Ala Leu Gly Val Gly Ser Arg
    55                  60                  65

gct cca cac ggc ggt atc ttc gtg gtc tgg gca atc gaa cca tgg tgg 355
Ala Pro His Gly Gly Ile Phe Val Val Trp Ala Ile Glu Pro Trp Trp
70                  75                  80                  85

ggc tgg ctc atc gca ctt gca gca ggc acc atc gtg tcc acc atc gtt 403
Gly Trp Leu Ile Ala Leu Ala Ala Gly Thr Ile Val Ser Thr Ile Val
                90                  95                  100

gtc atc gca ctg aag cag ttc tgg cca aac aag gcc gtc gct gca gaa 451
Val Ile Ala Leu Lys Gln Phe Trp Pro Asn Lys Ala Val Ala Ala Glu
            105                 110                 115

gtc gcg aag caa aaa gca caa caa gca gct gta aac gca taatcggacc 500
Val Ala Lys Gln Lys Ala Gln Gln Ala Ala Val Asn Ala
        120                 125                 130

ttgacccgat gtctggtcgg 520

<210> 8
<211> 130
<212> PRT
<213> Corynebacterium glutamicum

<400> 8
Leu Ser Ile Ala Thr Leu Leu Arg Lys Lys Leu Phe Thr Pro Ala Glu
1               5                   10                  15

Gln Glu Asn Gly Lys Ser Ser Trp Leu Leu Gly Leu Ala Phe Val Ser
            20                  25                  30

Glu Gly Ala Ile Pro Phe Ala Ala Ala Asp Pro Phe Arg Val Ile Pro
        35                  40                  45

Ala Met Met Ala Gly Gly Ala Thr Thr Gly Ala Ile Ser Met Ala Leu

50                  55                  60

Gly Val Gly Ser Arg Ala Pro His Gly Gly Ile Phe Val Val Trp Ala
65                  70                  75                  80

Ile Glu Pro Trp Trp Gly Trp Leu Ile Ala Leu Ala Ala Gly Thr Ile
                85                  90                  95

Val Ser Thr Ile Val Val Ile Ala Leu Lys Gln Phe Trp Pro Asn Lys
            100                 105                 110

Ala Val Ala Ala Glu Val Ala Lys Gln Lys Ala Gln Gln Ala Ala Val
        115                 120                 125

Asn Ala
    130

## Beschreibung

### Hintergrund der Erfindung

Bestimmte Produkte und Nebenprodukte von natürlich-vorkommenden Stoffwechselprozessen in Zellen werden in vielen Industriezweigen verwendet, einschließlich der Nahrungsmittel-, Futtermittel-, Kosmetik- und pharmazeutischen Industrie. Diese Moleküle, die gemeinsam als "Feinchemikalien" bezeichnet werden, umfassen organische Säuren, sowohl proteinogene als auch nicht-proteinogene Aminosäuren, Nukleotide und Nukleoside, Lipide und Fettsäuren, Diole, Kohlehydrate, aromatische Verbindungen, Vitamine und Cofaktoren sowie Enzyme. Ihre Produktion erfolgt am besten mittels Anzucht von Bakterien im Großmaßstab, die entwickelt wurden, um große Mengen des jeweils gewünschten Moleküls zu produzieren und sezernieren. Ein für diesen Zweck besonders geeigneter Organismus ist *Corynebacterium glutamicum,* ein gram-positives, nicht-pathogenes Bakterium. Über Stammselektion ist eine Reihe von Mutantenstämmen entwickelt worden, die ein Sortiment wünschenswerter Verbindungen produzieren. Die Auswahl von Stämmen, die hinsichtlich der Produktion eines bestimmten Moleküls verbessert sind, ist jedoch ein zeitaufwendiges und schwieriges Verfahren.

### Zusammenfassung der Erfindung

Diese Erfindung stellt neuartige Nukleinsäuremoleküle bereit, die sich zur Identifizierung oder Klassifizierung von *Corynebacterium glutamicum* oder verwandten Bakterienarten verwenden lassen. *C. glutamicum* ist ein gram-positives, aerobes Bakterium, das in der Industrie für die Produktion im Großmaßstab einer Reihe von Feinchemikalien, und auch zum Abbau von Kohlenwasserstoffen (bspw. beim Überlaufen von Rohöl) und zur Oxidation von Terpenoiden gemeinhin verwendet wird. Die Nukleinsäuremoleküle können daher zum Identifizieren von Mikroorganismen eingesetzt werden, die sich zur Produktion von Feinchemikalien, bspw. durch Fermentationsverfahren, verwenden lassen. *C*. *glutamicum* selbst ist zwar nichtpathogen, jedoch ist es mit anderen Corynebacterium-Arten, wie *Corynebacterium diphtheriae* (dem Erreger der Diphtherie) verwandt, die bedeutende Pathogene beim Menschen sind. Die Fähigkeit, das Vorhandensein von Corynebacterium-Arten zu identifizieren, kann daher auch eine signifikante klinische Bedeutung haben, z.B. bei diagnostischen Anwendungen. Diese Nukleinsäuremoleküle können zudem als Bezugspunkte zur Kartierung des *C*. *glutamicum-*Genoms oder von Genomen verwandter Organismen dienen.

Diese neuen Nukleinsäuremoleküle codieren Proteine, die hier als Phosphoenolpyruvat:Zucker-Phosphotransferasesystem-(PTS)-Proteine bezeichnet werden. Diese PTS-Proteine können bspw. energiereiche kohlenstoffhaltige Moleküle, wie Glucose, in *C. glutamicum* transportieren oder in diesen Mikroorganismen an der intrazellulären Signaltransduktion teilnehmen. Aufgrund der Verfügbarkeit von Klonierungsvektoren zur Verwendung in *Corynebacterium glutamicum,* wie bspw. offenbart in Sinskey et al., US-Patent Nr. 4 649 119, und Techniken zur genetischen Manipulation von *C. glutamicum* und den verwandten *Brevibacterium*-Arten (z.B. Lactofermentum) Yoshihama et al., J. Bacteriol. 162 (1985) 591-597; Katsumata et al., J. Bacteriol. 159 (1984) 306-311; und Santamaria et al. J. Gen. Microbiol. 130 (1984) 2237-2246), lassen sich die erfindungsgemäßen Nukleinsäuremoleküle zur genetischen Manipulation dieses Organismus verwenden, um es als Produzenten von einer oder mehreren Feinchemikalien besser und effizienter zu machen. Die erfindungsgemäßen PTS-Moleküle lassen sich so modifizieren, daß die Ausbeute, Produktion und/oder die Effizienz der Produktion von einer oder mehreren Feinchemikalien verbessert wird. Modifiziert man bspw. ein an der Glucose-Aufnahme beteiligtes PTS-Protein derart, daß seine Aktivität optimiert ist, kann die Menge der aufgenommenen Glucose oder die Geschwindigkeit, mit der die Glucose in die Zelle befördert wird, erhöht werden. Der Abbau von Glucose und anderen Zuckern in der Zelle liefert Energie, mit der sich energetisch ungünstige biochemische Reaktionen antreiben lassen, wie z.B. solche, die an der Biosynthese von Feinchemikalien beteiligt sind. Dieser Abbau stellt zudem Zwischen- und Vorstufen-Moleküle bereit, die für die Biosynthese bestimmter Feinchemikalien nötig sind, wie Aminosäuren, Vitamine und Cofaktoren. Durch Erhöhen der Menge an intrazellulären energiereichen Kohlenstoff-Molekülen über die Modifikation der erfindungsgemäßen PTS-Moleküle läßt sich daher sowohl die Energie, die zur Durchführung der für die Produktion einer oder mehrerer Feinchemikalien nötigen Stoffwechselwege verfügbar ist, als auch der intrazelluläre Pool an Metaboliten, die für diese Produktion nötig sind, vergrößern.

Die erfindungsgemäßen PTS-Moleküle können ferner an einem oder mehreren intrazellulären Signaltransduktionswegen beteiligt sein, die die Ausbeuten und/oder die Geschwindigkeit der Produktion einer oder mehrerer Feinchemikalien von *C. glutamicum* beeinflussen. Proteine, die bspw. für den Import von einem oder mehreren Zuckern aus dem extrazellulären Medium nötig sind (bspw. Hpr, Enzym I, oder ein Bestandteil des Enzym-II-Komplexes), werden bei Vorhandensein einer hinreichenden Menge Zucker in der Zelle häufig posttranslational modifiziert, so daß sie diesen Zucker nicht mehr importieren können. Die Zuckermenge, bei der das Transportsystem abgeschaltet wird, reicht zwar zur Aufrechterhaltung der normalen Zellfunktionen aus, jedoch schränkt sie die Überproduktion der gewünschten Feinchemikalie ein. Es empfiehlt sich daher, die erfindungsgemäßen PTS-Proteine zu modifizieren, so daß sie auf eine solche negative Regulation nicht mehr ansprechen. Dadurch lassen sich höhere intrazelluläre Konzentrationen eines oder mehrerer Zucker und durch Extension eine effizientere Produktion oder höhere Ausbeuten von einer oder mehreren Feinchemikalien aus Organismen erzielen, die diese mutanten PTS-Proteine enthalten.

Diese Erfindung stellt neue Nukleinsäuremoleküle bereit, die die hier als Phosphoenolpyruvat:zucker-Phosphotransferasesystem-(PTS) -Proteine bezeichneten Proteine codieren, welche bspw. am Import energiereicher Kohlenstoff-Moleküle (z.B. Glucose, Fructose oder Saccharose) in *C*. *glutamicum* und/oder an einem oder mehreren intrazellulären Signaltransduktionswegen von *C*. *glutamicum* beteiligt sein können. Nukleinsäuremoleküle, die ein PTS-Protein codieren, werden hier als PTS-Nukleinsäuremoleküle bezeichnet. Bei einer bevorzugten Ausführungsform ist das PTS-Protein am Import von energiereichen Kohlenstoff-Molekülen (z.B. Glucose, Fructose oder Saccharose) in *C. glutamicum* beteiligt und kann an einem oder mehreren intrazellulären Signaltransduktionswegen von *C. glutamicum* beteiligt sein. Beispiele für solche Proteine sind diejenigen, die von den in Tabelle 1 angegebenen Genen codiert werden.

Ein Aspekt der Erfindung betrifft folglich isolierte Nukleinsäuremoleküle (bspw. cDNAs), umfassend eine Nukleotidsequenz, die ein PTS-Protein oder biologisch aktive Abschnitte davon codiert, sowie Nukleinsäurefragmente, die sich als Primer oder Hybridisierungssonden zum Nachweisen oder zur Amplifikation von PTScodierender Nukleinsäure (bspw. DNA oder mRNA) eignen. Bei besonders bevorzugten Ausführungsformen umfaßt das isolierte Nukleinsäuremolekül eine der in Anhang A aufgeführten Nukleotidsequenzen oder den codierenden Bereich oder ein Komplement davon. In anderen bevorzugten Ausführungsformen codiert das isolierte Nukleinsäuremolekül eine der in Anhang B aufgeführten Aminosäuresequenzen. Die bevorzugten erfindungsgemäßen PTS-Proteine besitzen ebenfalls vorzugsweise mindestens eine der hier beschriebenen PTS-Aktivitäten.

Als Anhang A werden im folgenden die Nukleinsäuresequenzen des Sequenzprotokolls zusammen mit den in Tabelle 1 beschriebenen Sequenzveränderungen an der jeweiligen Position definiert.

Als Anhang B werden im folgenden die Polypeptidsequenzen des Sequenzprotokolls zusammen mit den in Tabelle 1 beschriebenen Sequenzveränderungen an der jeweiligen Position definiert.

Bei einer weiteren Ausführungsform ist das isolierte Nukleinsäuremolekül mindestens 15 Nukleotide lang und hybridisiert unter stringenten Bedingungen an ein Nukleinsäuremolekül, das eine Nukleotidsequenz aus Anhang A umfaßt. Das isolierte Nukleinsäuremolekül entspricht vorzugsweise einem natürlich vorkommenden Nukleinsäuremolekül. Die isolierte Nukleinsäure codiert stärker bevorzugt ein natürlich vorkommendes *C. glutamicum*-PTS-Protein oder einen biologisch aktiven Abschnitt davon.

Ein weiterer Aspekt der Erfindung betrifft Vektoren, bspw. rekombinante Expressionsvektoren, die die erfindungsgemäßen Nukleinsäuremoleküle enthalten, und Wirtszellen, in die diese Vektoren eingebracht worden sind. Bei einer Ausführungsform wird zur Herstellung eines PTS-Proteins eine Wirtszelle verwendet, die in einem geeigneten Medium gezüchtet wird. Das PTS-Protein kann dann aus dem Medium oder der Wirtszelle isoliert werden.

Ein weiterer Aspekt der Erfindung betrifft einen genetisch veränderten Mikroorganismus, bei dem ein PTS-Gen eingebracht oder verändert worden ist. Das Genom des Mikroorganismus ist bei einer Ausführungsform durch Einbringen mindestens eines erfindungsgemäßen Nukleinsäuremoleküls verändert worden, das die mutierte PTS-Sequenz als Transgen codiert. Bei einer anderen Ausführungsform ist ein endogenes PTS-Gen im Genom des Mikroorganismus durch homologe Rekombination mit einem veränderten PTS-Gen verändert, z.B. funktionell disruptiert, worden. Der Mikroorganismus gehört bei einer bevorzugten Ausführungsform zur Gattung *Corynebacterium* oder *Brevibacterium,* wobei *Corynebacterium glutamicum* besonders bevorzugt ist. Der Mikroorganismus wird in einer bevorzugten Ausführungsform auch zur Herstellung einer gewünschten Verbindung, wie einer Aminosäure, besonders bevorzugt Lysin, verwendet.

Eine weitere bevorzugte Ausführungsform sind Wirtszellen, die mehr als eine der in Anhang A beschriebenen Nukleinsäuremoleküle besitzen. Solche Wirtszellen lassen sich auf verschiedene dem Fachmann bekannte Wege herstellen. Beispielsweise können sie durch Vektoren, die mehrere der erfindungsgemäßen Nukleinsäuremoleküle tragen, transfiziert werden. Es ist aber auch möglich mit einem Vektor jeweils ein erfindungsgemäßes Nukleinsäuremolekül in die Wirtszelle einzubringen und deshalb mehrere Vektoren entweder gleichzeitig oder zeitlich abgestuft einzusetzen. Es können somit Wirtszellen konstruiert werden, die zahlreiche, bis zu mehreren Hundert der erfindungsgemäßen Nukleinsäuresequenzen tragen. Durch eine solche Akkumulation lassen sich häufig überadditive Effekte auf die Wirtszelle hinsichtlich der Feinchemikalien-Produktivität erzielen.

Ein weiterer Aspekt der Erfindung betrifft ein isoliertes PTS-Protein oder einen Abschnitt, bspw. einen biologisch aktiven Abschnitt davon. Das isolierte PTS-Protein oder sein Abschnitt kann in einer bevorzugten Ausführungsform am Import von energiereichen Kohlenstoff-Molekülen (bspw. Glucose, Fructose oder Saccharose) in *C*. *glutamicum* und zudem an einem oder mehreren intrazellulären Signaltransduktionswegen von *C. glutamicum* beteiligt sein. Bei einer weiteren bevorzugten Ausführungsform ist das isolierte PTS-Protein oder ein Abschnitt davon hinreichend homolog zu einer Aminosäuresequenz von Anhang B, so daß das Protein oder sein Abschnitt weiterhin am Import von energiereichen Kohlenstoff-Molekülen (bspw. Glucose, Fructose oder Saccharose) in *C*. *glutamicum* und/oder an einem oder mehreren intrazellulären Signaltransduktionswegen von *C. glutamicum* teilnehmen kann.

Die Erfindung betrifft zudem ein isoliertes PTS-Proteinpräparat. Das PTS-Protein umfaßt bei bevorzugten Ausführungsformen eine Aminosäuresequenz aus Anhang B. Bei einer weiteren bevorzugten Ausführungsform betrifft die Erfindung ein isoliertes Vollängenprotein, das zu einer vollständigen Aminosäuresequenz aus Anhang B (welche von einem offenen Leseraster in Anhang A codiert wird) im wesentlichen homolog ist.

Das PTS-Polypeptid oder ein biologisch aktiver Abschnitt davon kann mit einem Nicht-PTS-Polypeptid funktionsfähig verbunden werden, damit ein Fusionsprotein entsteht. Dieses Fusionsprotein hat bei bevorzugten Ausführungsformen eine andere Aktivität als das PTS-Protein allein und ergibt bei anderen bevorzugten Ausführungsformen erhöhte Ausbeuten, eine erhöhte Produktion und/oder Effizienz der Produktion einer gewünschten Feinchemikalie aus *C. glutamicum*. Die Integration dieses Fusionsproteins in eine Wirtszelle moduliert bei besonders bevorzugten Ausführungsformen die Produktion einer gewünschten Verbindung von der Zelle.

Ein weiterer Aspekt der Erfindung betrifft ein Verfahren zur Herstellung einer Feinchemikalie. Das Verfahren sieht die Anzucht einer Zelle vor, die einen Vektor enthält, der die Expression eines erfindungsgemäßen PTS-Nukleinsäuremoleküls bewirkt, so daß eine Feinchemikalie produziert wird. Dieses Verfahren umfaßt bei einer bevorzugten Ausführungsform zudem den Schritt der Gewinnung einer Zelle, die einen solchen Vektor enthält, wobei die Zelle mit einem Vektor transfiziert ist, der die Expression einer PTS-Nukleinsäure bewirkt. Dieses Verfahren umfaßt bei einer weiteren bevorzugten Ausführungsform zudem den Schritt, bei dem die Feinchemikalie aus der Kultur gewonnen wird. Die Zelle gehört bei einer bevorzugten Ausführungsform zur Gattung *Corynebacterium* oder *Brevibacterium.*

Ein weiterer Aspekt der Erfindung betrifft Verfahren zur Modulation der Produktion eines Moleküls aus einem Mikroorganismus. Diese Verfahren umfassen das Zusammenbringen der Zelle mit einer Substanz, die die PTS-Proteinaktivität oder die PTS-Nukleinsäure-Expression moduliert, so daß eine zellassoziierte Aktivität verglichen mit der gleichen Aktivität bei Fehlen der Substanz verändert wird. Die Zelle wird bei einer bevorzugten Ausführungsform hinsichtlich der Aufnahme eines oder mehrerer Zucker moduliert, so daß die Ausbeuten oder die Geschwindigkeit der Produktion einer gewünschten Feinchemikalie durch diesen Mikroorganismus verbessert wird. Die Substanz, die die PTS-Proteinaktivität moduliert, stimuliert bspw. die PTS-Proteinaktivität oder die PTS-Nukleinsäure-Expression. Beispiele von Substanzen, die die PTS-Proteinaktivität oder die PTS-Nukleinsäureexpression stimulieren, umfassen kleine Moleküle, aktive PTS-Proteine und Nukleinsäuren, die PTS-Proteine codieren und in die Zelle eingebracht worden sind. Beispiele von Substanzen, die die PTS-Aktivität oder -Expression hemmen, umfassen kleine Moleküle und Antisense-PTS-Nukleinsäuremoleküle.

Ein weiterer Aspekt der Erfindung betrifft Verfahren zur Modulation der Ausbeuten einer gewünschten Verbindung aus einer Zelle, umfassend das Einbringen eines PTS-Wildtyp- oder -Mutantengens in eine Zelle, das entweder auf einem gesonderten Plasmid bleibt oder in das Genom der Wirtszelle integriert wird. Die Integration in das Genom kann zufallsgemäß oder durch homologe Rekombination erfolgen, so daß das native Gen durch die integrierte Kopie ersetzt wird, was die Produktion der gewünschten Verbindung aus der zu modulierenden Zelle hervorruft. Diese Ausbeuten sind bei einer bevorzugten Ausführungsform erhöht. Bei einer weiteren bevorzugten Ausführungsform ist die Chemikalie eine Feinchemikalie, die in einer besonders bevorzugten Ausführungsform eine Aminosäure ist. Diese Aminosäure ist in einer besonders bevorzugten Ausführungsform L-Lysin.

### Eingehende Beschreibung der Erfindung

Die vorliegende Erfindung stellt PTS-Nukleinsäure- und -Protein-moleküle bereit, die an der Aufnahme energiereicher Kohlenstoff-Moleküle (bspw. Saccharose, Fructose oder Glucose) in *C. glutamicum* sind sowie auch an intrazellulären Signaltransduktionswegen in diesem Mikroorganismus beteiligt sein können. Die erfindungsgemäßen Moleküle lassen sich bei der Modulation der Produktion von Feinchemikalien von Mikroorganismen verwenden. Diese Modulation kann auf erhöhten intrazellulären Spiegeln energiereicher Moleküle beruhen, die zur Erzeugung von bspw. ATP, GTP und anderen Molekülen, benötigt werden, die zum Antreiben energetisch ungünstiger biochemischer Reaktionen in der Zelle, bspw. der Biosynthese einer Feinchemikalie, verwendet werden. Diese Modulation der Feinchemikalien-Produktion kann auch auf der Tatsache beruhen, daß die Abbauprodukte vieler Zucker als Zwischenprodukte oder Vorstufen für andere Biosynthesewege dienen, einschließlich denen bestimmter Feinchemikalien. Ferner sind PTS-Proteine bekanntlich an bestimmten intrazellulären Signaltransduktionswegen beteiligt, die für einen oder mehrere Stoffwechselwege von Feinchemikalien regulatorische Aktivität aufweisen können; durch Manipulation dieser PTS-Proteine kann man daher einen Feinchemikalien-Biosyntheseweg aktivieren oder einen Feinchemikalien-Abbauweg reprimieren. Die erfindungsgemäßen Aspekte werden nachstehend weiter erläutert.

### I. Feinchemikalien

Der Begriff "Feinchemikalie" ist im Fachgebiet bekannt und beinhaltet Moleküle, die von einem Organismus produziert werden und in verschiedenen Industriezweigen Anwendungen finden, wie bspw., jedoch nicht beschränkt auf die pharmazeutische Industrie, die Landwirtschafts-, und Kosmetik-Industrie. Diese Verbindungen umfassen organische Säuren, wie Weinsäure, Itaconsäure und Diaminopimelinsäure, sowohl proteinogene als auch nicht-proteinogene Aminosäuren, Purin- und Pyrimidinbasen, Nukleoside und Nukleotide (wie bspw. beschrieben in Kuninaka, A. (1996) Nucleotides and related compounds, S. 561-612, in Biotechnology Bd. 6, Rehm et al., Hrsg. VCH: Weinheim und den darin enthaltenen Zitaten), Lipide, gesättigte und ungesättigte Fettsäuren (bspw. Arachidonsäure), Diole (bspw. Propandiol und Butandiol), Kohlenhydrate (bspw. Hyaluronsäure und Trehalose), aromatische Verbindungen (bspw. aromatische Amine, Vanillin und Indigo), Vitamine und Cofaktoren (wie beschrieben in Ullmann's Encyclopedia of Industrial Chemistry, Bd. A27, "Vitamins", S. 443-613 (1996) VCH: Weinheim und den darin enthaltenen Zitaten; und Ong, A.S., Niki, E. und Packer, L. (1995) "Nutrition, Lipids, Health and Disease" Proceedings of the UNESCO/Confederation of Scientific and Technological Associations in Malaysia and the Society for Free Radical Research - Asien, abgehalten am 1.-3. Sept. 1994 in Penang, Malysia, AOCS Press (1995)), Enzyme und sämtliche anderen von Gutcho (1983) in Chemicals by Fermentation, Noyes Data Corporation, ISBN: 0818805086 und den darin angegebenen Literaturstellen, beschriebenen Chemikalien. Der Metabolismus und die Verwendungen bestimmter Feinchemikalien sind nachstehend weiter erläutert.

### A. Aminosäure-Metabolismus und Verwendungen

Die Aminosäuren umfassen die grundlegenden Struktureinheiten sämtlicher Proteine und sind somit für die normalen Zellfunktionen essentiell. Der Begriff "Aminosäure" ist im Fachgebiet bekannt. Die proteinogenen Aminosäuren, von denen es 20 Arten gibt, dienen als Struktureinheiten für Proteine, in denen sie über Peptidbindungen miteinander verknüpft sind, wohingegen die nichtproteinogenen Aminosäuren (von denen Hunderte bekannt sind) gewöhnlich nicht in Proteinen vorkommen (siehe Ullmann's Encyclopedia of Industrial Chemistry, Bd. A2, S. 57-97 VCH: Weinheim (1985)). Die Aminosäuren können in der D- oder L-Konfiguration vorliegen, obwohl L-Aminosäuren gewöhnlich der einzige Typ sind, den man in natürlich vorkommenden Proteinen vorfindet. Biosynthese- und Abbauwege von jeder der 20 proteinogenen Aminosäuren sind sowohl bei prokaryotischen als auch eukaryotischen Zellen gut charakterisiert (siehe bspw. Stryer, L. Biochemistry, 3. Auflage, S. 578-590 (1988)). Die "essentiellen" Aminosäuren (Histidin, Isoleucin, Leucin, Lysin, Methionin, Phenylalanin, Threonin, Tryptophan und Valin), so bezeichnet, da sie aufgrund der Komplexität ihrer Biosynthese mit der Ernährung aufgenommen werden müssen, werden durch einfache Biosyntheseswege in die übrigen 11 "nichtessentiellen" Aminosäuren (Alanin, Arginin, Asparagin, Aspartat, Cystein, Glutamat, Glutamin, Glycin, Prolin, Serin und Tyrosin) umgewandelt. Höhere Tiere besitzen die Fähigkeit, einige dieser Aminosäuren zu synthetisieren, jedoch müssen die essentiellen Aminosäuren mit der Nahrung aufgenommen werden, damit eine normale Proteinsynthese stattfindet.

Abgesehen von ihrer Funktion bei der Proteinbiosynthese sind diese Aminosäuren interessante Chemikalien an sich, und man hat entdeckt, daß viele bei verschiedenen Anwendungen in der Nahrungsmittel-, Futter-, Chemie-, Kosmetik-, Landwirtschafts- und pharmazeutischen Industrie zum Einsatz kommen. Lysin ist nicht nur für die Ernährung des Menschen eine wichtige Aminosäure, sondern auch für monogastrische Tiere, wie Geflügel und Schweine. Glutamat wird am häufigsten als Geschmacksadditiv (Mononatriumglutamat, MSG) sowie weithin in der Nahrungsmittelindustrie verwendet, wie auch Aspartat, Phenylalanin, Glycin und Cystein. Glycin, L-Methionin und Tryptophan werden sämtlich in der pharmazeutischen Industrie verwendet. Glutamin, Valin, Leucin, Isoleucin, Histidin, Arginin, Prolin, Serin und Alanin werden in der pharmazeutischen Industrie und der Kosmetik-industrie verwendet. Threonin, Tryptophan und D-/L-Methionin sind weitverbreitete Futtermittelzusätze (Leuchtenberger, W. (1996) Amino acids - technical production and use, S. 466-502 in Rehm et al., (Hrsg.) Biotechnology Bd. 6, Kapitel 14a, VCH: Weinheim). Man hat entdeckt, daß sich diese Aminosäuren außerdem als Vorstufen für die Synthese von synthetischen Aminosäuren und Proteinen, wie N-Acetylcystein, S-Carboxymethyl-L-cystein, (S)-5-Hydroxytryptophan und anderen, in Ullmann's Encyclopedia of Industrial Chemistry, Bd. A2, S. 57-97, VCH, Weinheim, 1985 beschriebenen Substanzen eignen.

Die Biosynthese dieser natürlichen Aminosäuren in Organismen, die sie produzieren können, bspw. Bakterien, ist gut charakterisiert worden (für einen Überblick der bakteriellen Aminosäure-Biosynthese und ihrer Regulation, s. Umbarger, H.E. (1978) Ann. Rev. Biochem. 47: 533 - 606). Glutamat wird durch reduktive Aminierung von α-Ketoglutarat, einem Zwischenprodukt im CitronensäureZyklus, synthetisiert. Glutamin, Prolin und Arginin werden jeweils nacheinander aus Glutamat erzeugt. Die Biosynthese von Serin erfolgt in einem Dreischritt-Verfahren und beginnt mit 3-Phosphoglycerat (einem Zwischenprodukt bei der Glykolyse), und ergibt nach Oxidations-, Transaminierungs- und Hydrolyseschritten diese Aminosäure. Cystein und Glycin werden jeweils aus Serin produziert, und zwar die erstere durch Kondensation von Homocystein mit Serin, und die letztere durch Übertragung des Seitenketten-β-Kohlenstoffatoms auf Tetrahydrofolat, in einer durch Serintranshydroxymethylase katalysierten Reaktion. Phenylalanin und Tyrosin werden aus den Vorstufen des Glycolyse- und Pentosephosphatweges, Erythrose-4-phosphat und Phosphoenolpyruvat in einem 9-Schritt-Biosyntheseweg synthetisiert, der sich nur in den letzten beiden Schritten nach der Synthese von Prephenat unterscheidet. Tryptophan wird ebenfalls aus diesen beiden Ausgangsmolekülen produziert, jedoch erfolgt dessen Synthese in einem 11-Schritt-Weg. Tyrosin läßt sich in einer durch Phenylalaninhydroxylase katalysierten Reaktion auch aus Phenylalanin herstellen. Alanin, Valin und Leucin sind jeweils Biosyntheseprodukte aus Pyruvat, dem Endprodukt der Glykolyse. Aspartat wird aus Oxalacetat, einem Zwischenprodukt des Citratzyklus, gebildet. Asparagin, Methionin, Threonin und Lysin werden jeweils durch Umwandlung von Aspartat produziert. Isoleucin wird aus Threonin gebildet. In einem komplexen 9-Schritt-Weg erfolgt die Bildung von Histidin aus 5-Phosphoribosyl-l-pyrophosphat, einem aktivierten Zucker.

Aminosäuren, deren Menge den Proteinbiosynthesebedarf der Zelle übersteigt, können nicht gespeichert werden, und werden stattdessen abgebaut, so daß Zwischenprodukte für die Haupt-Stoffwechselwege der Zelle bereitgestellt werden (für einen Überblick siehe Stryer, L., Biochemistry, 3. Aufl. Kap. 21 "Amino Acid Degradation and the Urea Cycle" ; S 495-516 (1988)). Die Zelle ist zwar in der Lage, ungewünschte Aminosäuren in nützliche Stoffwechsel-Zwischenprodukte umzuwandeln, jedoch ist die Aminosäureproduktion hinsichtlich der Energie, der Vorstufenmoleküle und der für ihre Synthese nötigen Enzyme aufwendig. Es überrascht daher nicht, daß die Aminosäure-Biosynthese durch Feedback-Hemmung reguliert wird, wobei das Vorliegen einer bestimmten Aminosäure ihre eigene Produktion verlangsamt oder ganz beendet (für einen Überblick über den Rückkopplungs-Mechanismus bei Aminosäure-Biosynthesewegen, siehe Stryer, L., Biochemistry, 3. Aufl., Kap. 24, "Biosynthesis of Amino Acids and Heme", S. 575-600 (1988)). Der Ausstoß einer bestimmten Aminosäure wird daher durch die Menge dieser Aminosäure in der Zelle eingeschränkt.

### B. Vitamine, Cofaktoren und Nutrazeutika-Metabolismus sowie Verwendungen

Vitamine, Cofaktoren und Nutrazeutika umfassen eine weitere Gruppe von Molekülen. Höhere Tiere haben die Fähigkeit verloren, diese zu synthetisieren und müssen sie somit aufnehmen, obwohl sie leicht durch andere Organismen, wie Bakterien, synthetisiert werden. Diese Moleküle sind entweder biologisch aktive Moleküle an sich oder Vorstufen von biologisch aktiven Substanzen, die als Elektronenträger oder Zwischenprodukte bei einer Reihe von Stoffwechselwegen dienen. Diese Verbindungen haben neben ihrem Nährwert auch einen signifikanten industriellen Wert als Farbstoffe, Antioxidantien und Katalysatoren oder andere Verarbeitungs-Hilfsstoffe. (Für einen Überblick über die Struktur, Aktivität und die industriellen Anwendungen dieser Verbindungen siehe bspw. Ullmann's Encyclopedia of Industrial Chemistry, "Vitamins", Bd. A27, S. 443-613, VCH: Weinheim, 1996). Der Begriff "Vitamin" ist im Fachgebiet bekannt und umfaßt Nährstoffe, die von einem Organismus für eine normale Funktion benötigt werden, jedoch nicht von diesem Organismus selbst synthetisiert werden können. Die Gruppe der Vitamine kann Cofaktoren und nutrazeutische Verbindungen umfassen. Der Begriff "Cofaktor" umfaßt nicht-proteinartige Verbindungen, die für das Auftreten einer normalen Enzymaktivität nötig sind. Diese Verbindungen können organisch oder anorganisch sein; die erfindungsgemäßen Cofaktor-Moleküle sind vorzugsweise organisch. Der Begriff "Nutrazeutikum" umfaßt Nahrungsmittelzusätze, die bei Pflanzen und Tieren, insbesondere dem Menschen, gesundheitsfördernd sind. Beispiele solcher Moleküle sind Vitamine, Antioxidantien und ebenfalls bestimmte Lipide (z.B. mehrfach ungesättigte Fettsäuren).

Die Biosynthese dieser Moleküle in Organismen, die zu ihrer Produktion befähigt sind, wie Bakterien, ist umfassend charakterisiert worden (Ullmann's Encyclopedia of Industrial Chemistry, "Vitamins", Bd. A27, S. 443-613, VCH: Weinheim, 1996, Michal, G. (1999) Biochemical Pathways: An Atlas of Biochemistry and Molecular Biology, John Wiley & Sons; Ong, A.S., Niki, E. und Packer, L. (1995) "Nutrition, Lipids, Health and Disease" Proceedings of the UNESCO/Confederation of Scientific and Technological Associations in Malaysia and the Society for free Radical Research - Asien, abgehalten am 1.-3. Sept. 1994 in Penang, Malaysia, AOCS Press, Champaign, IL X, 374 S).

Thiamin (Vitamin B₁) wird durch chemisches Kuppeln von Pyrimidin und Thiazol-Einheiten gebildet. Riboflavin (Vitamin B₂) wird aus Guanosin-5'-triphosphat (GTP) und Ribose-5'-phosphat synthetisiert. Riboflavin wiederum wird zur Synthese von Flavinmononukleotid (FMN) und Flavinadenindinukleotid (FAD) eingesetzt. Die Familie von Verbindungen, die gemeinsam als "Vitamin B6" bezeichnet werden (bspw. Pyridoxin, Pyridoxamin, Pyridoxal-5'-phosphat und das kommerziell verwendete Pyridoxinhydrochlorid), sind alle Derivate der gemeinsamen Struktureinheit 5-Hydroxy-6-methylpyridin. Panthothenat (Pantothensäure, R-(+)-N-(2,4-Dihydroxy-3,3-dimethyl-1-oxobutyl)-β-alanin) kann entweder durch chemische Synthese oder durch Fermentation hergestellt werden. Die letzten Schritte bei der Pantothenat-Biosynthese bestehen aus der ATPgetriebenen Kondensation von β-Alanin und Pantoinsäure. Die für die Biosyntheseschritte für die Umwandlung in Pantoinsäure, in β-Alanin und zur Kondensation in Pantothensäure verantwortlichen Enzyme sind bekannt. Die metabolisch aktive Form von Pantothenat ist Coenzym A, dessen Biosynthese über 5 enzymatische Schritte verläuft. Pantothenat, Pyridoxal-5'-phosphat, Cystein und ATP sind die Vorstufen von Coenzym A. Diese Enzyme katalysieren nicht nur die Bildung von Pantothenat, sondern auch die Produktion von (R)-Pantoinsäure, (R)-Pantolacton, (R)-Panthenol (Provitamin B₅), Pantethein (und seinen Derivaten) und Coenzym A.

Die Biosynthese von Biotin aus dem Vorstufenmolekül Pimeloyl-CoA in Mikroorganismen ist ausführlich untersucht worden, und man hat mehrere der beteiligten Gene identifiziert. Es hat sich herausgestellt, daß viele der entsprechenden Proteine an der Fe-Cluster-Synthese beteiligt sind und zu der Klasse der nifS-Proteine gehören. Die Liponsäure wird von der Octanonsäure abgeleitet und dient als Coenzym beim Energie-Metabolismus, wo sie Bestandteil des Pyruvatdehydrogenasekomplexes und des α-Ketoglutaratdehydrogenasekomplexes wird. Die Folate sind eine Gruppe von Substanzen, die alle von der Folsäure abgeleitet werden, die wiederum von L-Glutaminsäure, p-Aminobenzoesäure und 6-Methylpterin hergeleitet ist. Die Biosynthese der Folsäure und ihrer Derivate, ausgehend von den metabolischen Stoffwechselzwischenprodukten Guanosin-5'-triphosphat (GTP), L-Glutaminsäure und p-Aminobenzoesäure ist in bestimmten Mikroorganismen eingehend untersucht worden.

Corrinoide (wie die Cobalamine und insbesondere Vitamin B₁₂) und die Porphyrine gehören zu einer Gruppe von Chemikalien, die sich durch ein Tetrapyrrol-Ringsystem auszeichnen. Die Biosynthese von Vitamin B₁₂ ist hinreichend komplex, daß sie noch nicht vollständig charakterisiert worden ist, jedoch ist inzwischen ein Großteil der beteiligten Enzyme und Substrate bekannt. Nikotinsäure (Nikotinat) und Nikotinamid sind Pyridin-Derivate, die auch als "Niacin" bezeichnet werden. Niacin ist die Vorstufe der wichtigen Coenzyme NAD (Nikotinamidadenindinukleotid) und NADP (Nikotinamidadenindinukleotidphosphat) und ihrer reduzierten Formen.

Die Produktion dieser Verbindungen im Großmaßstab beruht größtenteils auf zellfreien chemischen Synthesen, obwohl einige dieser Chemikalien ebenfalls durch großangelegte Anzucht von Mikroorganismen produziert worden sind, wie Riboflavin, Vitamin B₆, Pantothenat und Biotin. Nur Vitamin B₁₂ wird aufgrund der Komplexität seiner Synthese lediglich durch Fermentation produziert. In-vitro-Verfahren erfordern einen erheblichen Aufwand an Materialien und zeit und häufig an hohen Kosten.

### C. Purin-, Pyrimidin-, Nukleosid- und Nukleotid-Metabolismus und Verwendungen

Gene für den Purin- und Pyrimidin-Stoffwechsel und ihre entsprechenden Proteine sind wichtige Ziele für die Therapie von Tumorerkrankungen und Virusinfektionen. Der Begriff "Purin" oder "Pyrimidin" umfaßt stickstoffhaltige Basen, die Bestandteil der Nukleinsäuren, Coenzyme und Nukleotide sind. Der Begriff "Nukleotid" beinhaltet die grundlegenden Struktureinheiten der Nukleinsäuremoleküle, die eine stickstoffhaltige Base, einen Pentose-Zucker (bei RNA ist der Zucker Ribose, bei DNA ist der Zucker D-Desoxyribose) und Phosphorsäure umfassen. Der Begriff "Nukleosid" umfaßt Moleküle, die als Vorstufen von Nukleotiden dienen, die aber im Gegensatz zu den Nukleotiden keine Phosphorsäureeinheit aufweisen. Durch Hemmen der Biosynthese dieser Moleküle oder ihrer Mobilisation zur Bildung von Nukleinsäuremolekülen ist es möglich, die RNA- und DNA-Synthese zu hemmen; wird diese Aktivität zielgerichtet bei kanzerogenen Zellen gehemmt, läßt sich die Teilungs- und Replikations-Fähigkeit von Tumorzellen hemmen.

Es gibt zudem Nukleotide, die keine Nukleinsäuremoleküle bilden, jedoch als Energiespeicher (d.h. AMP) oder als Coenzyme (d.h. FAD und NAD) dienen.

Mehrere Veröffentlichungen haben die Verwendung dieser Chemikalien für diese medizinischen Indikationen beschrieben, wobei der Purin- und/oder Pyrimidin-Metabolismus beeinflußt wird (bspw. Christopherson, R.I. und Lyons, S.D. (1990) "Potent inhibitors of de novo pyrimidine and purine biosynthesis as chemotherapeutic agents", Med. Res. Reviews 10: 505-548). Untersuchungen an Enzymen, die am Purin- und Pyrimidin-Metabolismus beteiligt sind, haben sich auf die Entwicklung neuer Medikamente konzentriert, die bspw. als Immunsuppressionsmittel oder Antiproliferantien verwendet werden können (Smith, J.L. "Enzymes in Nucleotide Synthesis" Curr. Opin. Struct. Biol. 5 (1995) 752-757; Biochem. Soc. Transact. 23 (1995) 877-902). Die Purin- und Pyrimidinbasen, Nukleoside und Nukleotide haben jedoch auch andere Einsatzmöglichkeiten: als Zwischenprodukte bei der Biosysnthese verschiedener Feinchemikalien (z.B. Thiamin, S-Adenosyl-methionin, Folate oder Riboflavin), als Energieträger für die Zelle (bspw. ATP oder GTP) und für Chemikalien selbst, werden gewöhnlich als Geschmacksverstärker verwendet (bspw. IMP oder GMP) oder für viele medizinische Anwendungen (siehe bspw. Kuninaka, A., (1996) "Nucleotides and Related Compounds in Biotechnology Bd. 6, Rehm et al., Hrsg. VCH: Weinheim, S. 561-612). Enzyme, die am Purin-, Pyrimidin-, Nukleosid- oder Nukleotid-Metabolismus beteiligt sind, dienen auch immer stärker als Ziele, gegen die Chemikalien für den Pflanzenschutz, einschließlich Fungiziden, Herbiziden und Insektiziden entwickelt werden.

Der Metabolismus dieser Verbindungen in Bakterien ist charakterisiert worden (für Übersichten siehe bspw. Zalkin, H. und Dixon, J.E. (1992) "De novo purin nucleotide biosynthesis" in Progress in Nucleic Acids Research and Molecular biology, Bd. 42, Academic Press, S. 259-287; und Michal, G. (1999) "Nucleotides and Nucleosides"; Kap. 8 in : Biochemical Pathways: An Atlas of Biochemistry and Molecular Biology, Wiley, New York). Der Purin-Metabolismus, das Objekt intesiver Forschung, ist für das normale Funktionieren der Zelle essentiell. Ein gestörter Purin-Metabolismus in höheren Tieren kann schwere Erkrankungen verursachen, bspw. Gicht. Die Purinnukleotide werden über eine Reihe von Schritten über die Zwischenverbindung Inosin-5'-phosphat (IMP) aus Ribose-5-phosphat synthetisiert, was zur Produktion von Guanosin-5'-monophosphat (GMP) oder Adenosin-5'-monophosphat (AMP) führt, aus denen sich die als Nukleotide verwendeten Triphosphatformen leicht herstellen lassen. Diese Verbindungen werden auch als Energiespeicher verwendet, so daß ihr Abbau Energie für viele verschiedene biochemische Prozesse in der Zelle liefert. Die Pyrimidinbiosynthese erfolgt über die Bildung von Uridin-5'-monophosphat (UMP) aus Ribose-5-phosphat. UMP wiederum wird in cytidin-5'-triphosphat (CTP) umgewandelt. Die Desoxyformen sämtlicher Nukleotide werden in einer Einschritt-Reduktionsreaktion aus der Diphosphat-Riboseform des Nukleotides zur Diphosphat-Desoxyriboseform des Nukleotides hergestellt. Nach der Phosphorylierung können diese Moleküle an der DNA-Synthese teilnehmen.

### D. Trehalose-Metabolismus und Verwendungen

Trehalose besteht aus zwei Glucosemolekülen, die über α,α-1,1-Bindung miteinander verknüpft sind. Sie wird gewöhnlich in der Nahrungsmittelindustrie als Süßstoff, als Additiv für getrocknete oder gefrorene Nahrungsmittel sowie in Getränken verwendet. Sie wird jedoch auch in der pharmazeutischen Industrie, der Kosmetik- und Biotechnologie-Industrie angewendet (s. bspw. Nishimoto et al., (1998) US-Patent Nr. 5 759 610; Singer, M.A. und Lindquist, S. Trends Biotech. 16 (1998) 460-467; Paiva, C.L.A. und Panek, A.D. Biotech Ann. Rev. 2 (1996) 293-314; und Shiosaka, M.J. Japan 172 (1997) 97-102). Trehalose wird durch Enzyme von vielen Mikroorganismen produziert und auf natürliche Weise in das umgebende Medium abgegeben, aus dem sie durch im Fachgebiet bekannte Verfahren gewonnen werden kann.

### II. Das Phosphoenolpyruvat:Zucker-Phosphotransferase-System

Die Fähigkeit von Zellen, zu wachsen und sich rasch in Kultur zu teilen ist zu einem hohen Grad vom Ausmaß abhängig, mit dem die Zellen energiereiche Moleküle aufnehmen und nutzen können, wie Glucose und andere Zucker. Es gibt unterschiedliche Transporterproteine zum Transport unterschiedlicher Kohlenstoffquellen in die Zelle, bspw. Transportproteine für Zucker, wie Glucose, Fructose, Mannose, Galactose, Ribose, Sorbose, Ribulose, Lactose, Maltose, Saccharose oder Raffinose, sowie auch Transportproteine für Stärke- oder zelluloseabbauprodukte. Andere Transportsysteme dienen dem Import von Alkoholen (z.B. Methanol oder Ethanol), Alkanen, Fettsäuren und organischen Säuren, wie Essigsäure oder Milchsäure. In Bakterien können Zucker durch eine Vielzahl von Mechanismen über die Zellmembran in die Zelle befördert werden. Neben dem Zucker-Protonen-Symport ist das bakterielle Phosphoenolpyruvat:Zucker-Phosphotransferase-System (PTS) eines der gebräuchlichsten Verfahren für die Zuckeraufnahme. Dieses System katalysiert nicht nur die Translokation (mit einhergehender Phosphorylierung) von Zuckern und Hexitolen, sondern reguliert auch den zellulären Metabolismus in Abhängigkeit der Verfügbarkeit von Kohlenhydraten. Diese PTS-Systeme sind in Bakterien ubiquitär, kommen aber nicht in Archebakterien oder Eukaryonten vor. Das PTS-System besteht funktionell aus zwei cytoplasmatischen Proteinen, Enzym I und HPr, und einer variablen Anzahl zuckerspezifischer integraler und peripherer Membran-Transportkomplexe (die jeweils als "Enzym II" mit einer zuckerspezifischen Hochstellung bezeichnet werden, z.B. Enzym II^{Glu}" für den glucosebindenen Enzym-II-Komplex). Enzyme II, die für Mono- Di- oder Oligosaccharide, wie Glucose, Fructose, Mannose, Galactose, Ribose, Sorbose, Ribulose, Lactose, Maltose, Saccharose, Raffinose und andere spezifisch sind, sind bekannt. Das Enzym I überträgt Phosphorylgruppen von Phosphoenolpyruvat (PEP) auf das Phosphoryl-Trägerprotein HPr. HPr überträgt dann die Phosphorylgruppen auf verschiedene Enzym-II-Transportkomplexe. Die Aminosäuresequenzen von Enzym I und HPr sind zwar in allen Bakterien recht ähnlich, jedoch lassen sich die Sequenzen für PTS-Transporter in strukturell unverwandte Familien unterteilen. Die Anzahl und die Homologie zwischen diesen Genen variiert von Bakterium zu Bakterium. Das E. coli-Genom codiert 38 unterschiedliche PTS-Proteine, von denen 33 Untereinheiten sind, die zu 22 verschiedenen Transportern gehören. Das M. genitalium-Genom enthält jeweils 1 Gen für Enzym I und HPr, und nur zwei Gene für PTS-Transporter. Die Genome von *T. palladium* und *C. trachomatis* enthalten Gene für Enzym-I- und HPr-ähnliche Proteine, nicht aber für PTS-Transporter.

Sämtliche PTS-Transporter bestehen aus drei funktionellen Einheiten IIA, IIB und IIC, die entweder als Proteinuntereinheiten in einem Komplex (z.B. IIA^{Glc}IICB^{Glc}) oder als Domänen einer einzigen Polypeptidkette (z.B. IICBA^{Glc}NAc) vorkommen. IIA und IIB übertragen nacheinander Phosphorylgruppen von HPr auf die transportierten Zucker. IIC enthält die Zuckerbindungsstelle und überspannt die Innenmembran sechs oder acht Mal. Die Zuckertranslokation ist mit der transienten Phosphorylierung der IIB-Domäne gekoppelt. Das Enzym I, HPr und IIA werden an den Histidinresten phosphoryliert, wohingegen die IIB-Untereinheiten je nach dem beteiligten Transporter entweder an den Cystein- oder den Histidinresten phosphoryliert sind. Die Phosphorylierung des importierten Zuckers hat den Vorteil, daß die Diffusion des Zuckers durch die Zellmembran zurück in das extrazelluläre Medium blockiert wird, da die geladene Phosphatgruppe den hydrophoben Kern der Membran nicht leicht durchqueren kann.

Einige PTS-Proteine spielen neben ihrer Funktion beim aktiven Transport von Zuckern eine Rolle bei der intrazellulären Signaltransduktion. Diese Untereinheiten regulieren ihre Ziele entweder allosterisch oder durch Phosphorylierung. Ihre regulatorische Aktivität variiert mit dem Ausmaß ihrer Phosphorylierung (d.h. dem Verhältnis der nicht-phosphorylierten zur phosphorylierten Form), das wiederum mit dem Verhältnis der zuckerabhängigen Dephosphorylierung und der Phosphoenolpyruvat-abhängigen Rephosphorylierung variiert. Beispiele einer solchen intrazellulären Regulation durch PTS-Proteine in *E. coli* umfassen die Hemmung von Glycerinkinase durch dephosphoryliertes IIA^{Glc}, und die Aktivierung der Adenylatcyclase durch die phosphorylierte Version dieses Proteins. Die HPr- und IIB-Domänen einiger Transporter in diesen Mikroorganismen regulieren die Genexpression durch reversible Phosphorylierung der Transkriptionsantiterminatoren. In gram-positiven Bakterien wird die Aktivität von HPr durch HPrspezifische Serinkinasen und Phosphatasen moduliert. Bspw. wirkt HPr, das an Serin 46 phosphoryliert ist, als Corepressor des Transkriptions-Repressors CcpA. Schließlich hat man entdeckt, daß unphosphoryliertes Enzym I die Sensorkinase CheA der bakteriellen Chemotaxis-Maschinerie hemmt, was eine direkte Verbindung zwischen der Zuckerbindung und den Transportsystemen des Bakteriums und solchen Systemen schafft, die die Bakterienbewegung bewirken (Sonenshein, A.L. et al., Hrsg. Bacillus subtilis und andere grampositive Bakterien. ASM: Washington, DC; Neidhard, F.C. et al., Hrsg. (1996) Escherichia coli und Salmonella. ASM Press Washington, DC; Lengeler et al., (1999) Biology of Prokaryotes, Abschnitt II, S. 68-87, Thieme-Verlag, Stuttgart).

### III. Erfindungsgemäße Elemente und Verfahren

Die vorliegende Erfindung beruht zumindest teilweise auf der Entdeckung von neuen Molekülen, die hier als PTS-Nukleinsäure- und -Protein-Moleküle bezeichnet werden und an der Aufnahme energiereicher Kohlenstoffmoleküle (z.B. Glucose, Saccharose und Fructose) in *C. glutamicum* beteiligt sind und auch an einem oder mehreren intrazellulären Signaltransduktionswegen in diesen Mikroorganismen beteiligt sein können. Bei einer Ausführungsform importieren die PTS-Moleküle energiereiche Kohlenstoffmoleküle in die Zelle, wo die durch ihren Abbau erzeugte Energie zum Antreiben energetisch weniger begünstigter biochemischer Reaktionen eingesetzt wird. Ihre Abbauprodukte können als Zwischenprodukte oder Vorstufen für eine Reihe anderer Stoffwechselwege eingesetzt werden. Bei einer anderen Ausführungsform können die PTS-Moleküle an einem oder mehreren intrazellulären Signaltransduktionswegen teilnehmen, wobei die Gegenwart einer modifizierten Form eines PTS-Moleküls (z.B. ein phosphoryliertes PTS-Protein) an einer Signaltransduktionskaskade teilnehmen kann, die einen oder mehrere Zellvorgänge reguliert. Die Aktivität der erfindungsgemäßen PTS-Moleküle hat bei einer bevorzugten Ausführungsform eine Auswirkung auf die Produktion einer gewünschten Feinchemikalie durch diesen Organismus. Bei einer besonders bevorzugten Ausführungsform haben die erfindungsgemäßen PTS-Moleküle eine modulierte Aktivität, so daß die Ausbeute, Produktion oder Effizienz der Produktion von einer oder mehreren Feinchemikalien aus *C*. *glutamicum* ebenfalls moduliert sind.

Der Begriff "PTS-Protein" oder "PTS-Polypeptid" umfaßt Proteine, die an der Aufnahme von einer oder mehreren energiereichen Kohlenstoffverbindungen (z.B. Mono-, Di- oder Oligosacchariden, wie Fructose, Mannose, Saccharose, Glucose, Raffinose, Galactose, Ribose, Lactose, Maltose und Ribulose) aus dem extrazellulären Medium ins Innere der Zelle beteiligt sind. Diese PTS-Proteine können ebenfalls am einem oder mehreren intrazellulären Signaltransduktionswegen beteiligt sein, wie bspw., jedoch nicht beschränkt auf, diejenigen, die die Aufnahme verschiedener Zucker in die Zelle bewirken. Beispiele für PTS-Proteine umfassen solche, die von den in Tabelle 1 und Anhang A aufgeführten PTS-Genen codiert werden. Für allgemeine Literaturstellen bezüglich des PTS-Systems siehe Stryer, L., (1988) Biochemistry. Kapitel 37: "Membrane Transport", W.H. Freeman: New York, S. 959-961; Darnell, J. et al. (1990) Molecular Cell Biology, Scientific American Books; New York, S. 552-553 und Michal, G. Hrsg. (1999) Biochemical Pathways: An Atlas of Biochemistry and Molecular Biology, Kapitel 15 "Special Bacterial Metabolism". Die Ausdrücke "PTS-Gen" oder "PTS-Nukleinsäuresequenz" umfassen Nukleinsäuresequenzen, die ein PTS-Protein codieren, das aus einem codierenden Bereich und entsprechenden untranslatierten 5'- und 3'-Sequenzbereichen besteht. Beispiele für PTS-Gene sind in Tabelle 1 aufgelistet. Die Begriffe "Produktion" oder "Produktivität" sind im Fachgebiet bekannt und beinhalten die Konzentration des Fermentationsproduktes (bspw. der gewünschten Feinchemikalie, die innerhalb einer festgelegten Zeitspanne und eines festgelegten Fermentationsvolumens gebildet werden (bspw. kg Produkt pro Std. pro 1). Der Begriff "Effizienz der Produktion" umfaßt die Zeit, die zur Erzielung einer bestimmten Produktionsmenge nötig ist (bspw. wie lange die Zelle zur Aufrichtung einer bestimmten Durchsatzrate einer Feinchemikalie benötigt). Der Begriff "Ausbeute" oder "Produkt/Kohlenstoff-Ausbeute" ist im Fachgebiet bekannt und umfaßt die Effizienz der Umwandlung der Kohlenstoffquelle in das Produkt (d.h. die Feinchemikalie). Dies wird bspw. gewöhnlich ausgedrückt als kg Produkt pro kg Kohlenstoffquelle. Durch Vergrößern der Ausbeute oder Produktion der Verbindung wird die Menge der gewonnenen Moleküle oder der geeigneten gewonnenen Moleküle dieser Verbindung in einer bestimmten Kulturmenge über einen festgelegten Zeitraum erhöht. Die Begriffe "Biosynthese" oder "Biosyntheseweg" sind im Fachgebiet bekannt und umfassen die Synthese einer Verbindung, vorzugsweise einer organischen Verbindung, durch eine Zelle aus Zwischenverbindungen, bspw. in einem Mehrschritt- oder stark regulierten Prozeß. Die Begriffe "Abbau" oder "Abbauweg" sind im Fachgebiet bekannt und umfassen die Spaltung einer Verbindung, vorzugsweise einer organischen Verbindung, durch eine Zelle in Abbauprodukte (allgemeiner gesagt, kleinere oder weniger komplexe Moleküle), bspw. in einem Mehrschritt- oder stark regulierten Prozeß. Der Begriff "Metabolismus" ist im Fachgebiet bekannt und umfaßt die Gesamtheit der biochemischen Reaktionen, die in einem Organismus stattfinden. Der Metabolismus einer bestimmten Verbindung (z.B. der Metabolismus einer Aminosäure, wie Glycin) umfaßt dann sämtliche Biosynthese-, Modifikations- und Abbauwege dieser Verbindung in der Zelle. Der Begriff "Transport" oder "Import" ist im Fachgebiet bekannt und umfaßt die erleichterte Bewegung eines oder mehrerer Moleküle über eine Zellmembran, durch die das Molekül ansonsten nicht gelangen kann.

Die erfindungsgemäßen PTS-Moleküle sind in einer anderen Ausführungsform befähigt, die Produktion eines gewünschten Moleküls, wie einer Feinchemikalie, in einem Mikroorganismus, wie *C. glutamicum,* zu modulieren. Mit Hilfe von Genrekombinationstechniken lassen sich ein oder mehrere erfindungsgemäße PTS-Proteine derart manipulieren, daß ihre Funktion moduliert ist. Bspw. kann ein Protein, das am PTS-vermittelten Import von Glucose beteiligt ist, so verändert werden, daß es optimale Aktivität aufweist, und das PTS-System für den Import von Glucose kann somit größere Mengen Glucose in die Zelle befördern. Glucosemoleküle werden nicht nur als Energiequelle für energetisch ungünstige biochemische Reaktionen, wie die Biosynthese von Feinchemikalien verwendet, sondern auch als Vorstufen und Zwischenprodukte bei einer Reihe von Feinchemikalien-Biosynthesewegen (bspw. wird Serin aus 3-Phosphoglycerat synthetisiert). In jedem Fall läßt sich die Gesamtausbeute oder die Produktionsrate einer dieser gewünschten Feinchemikalien erhöhen, und zwar durch Erhöhen der Energie, die verfügbar ist, damit diese Produktion stattfindet, oder durch Vergrößern der Verfügbarkeit der Verbindungen, die nötig sind, damit diese Produktion stattfindet.

Es ist zudem bekannt, daß viele PTS-Proteine eine Schlüsselrolle bei intrazellulären Signaltransduktionswegen spielen, die den Zellmetabolismus und die Zuckeraufnahme regulieren, indem sie die Verfügbarkeit von Kohlenstoffquellen aufrechterhalten. Man weiß bspw., daß ein erhöhter intrazellulärer Spiegel von Fructose-1,6-bisphosphat (einer bei der Glycolyse erzeugten Verbindung) die Phosphorylierung eines Serinrestes von HPr bewirkt, was verhindert, daß dieses Protein in einem PTS-Zucker-Transportprozeß als Phosphoryldonor dient, wodurch die weitere Zuckeraufnahme blockiert wird. Mutagenisiert man HPr derart, daß dieser Serinrest nicht phosphoryliert werden kann, kann man HPr konstitutiv aktivieren, und somit den Zuckertransport in die Zelle verstärken, was wiederum größere intrazelluläre Energiespeicher und Zwischenprodukt/Vorstufen-Moleküle für die Biosynthese von einer oder mehreren gewünschten Feinchemikalien gewährleistet.

Als Ausgangspunkt zur Herstellung der erfindungsgemäßen Nukleinsäuresequenzen eignet sich das Genom eines *Corynebacterium glutamicum*-Stammes, der von der American Type Culture Collection unter der Bezeichnung ATCC 13032 erhältlich ist.

Von diesen Nukleinsäuresequenzen lassen sich durch die in Tabelle 1 bezeichneten Veränderungen die erfindungsgemäßen Nukleinsäuresequenzen mit üblichen Verfahren herstellen.

Das erfindungsgemäße PTS-Protein oder ein biologisch aktiver Abschnitt oder Fragmente davon können am Transport von energiereichen kohlenstoffhaltigen Molekülen, wie Glucose, in *C. glutamicum* oder an einer intrazellulären Signaltransduktion in diesem Mikroorganismus beteiligt sein, oder können eine oder mehrere der in Tabelle 1 aufgeführten Aktivitäten aufweisen.

In den nachstehenden Unterabschnitten sind verschiedene Aspekte der Erfindung ausführlicher beschrieben:

### A. Isolierte Nukleinsäuremoleküle

Ein Aspekt der Erfindung betrifft isolierte Nukleinsäuremoleküle, die PTS-Polypeptide oder biologisch aktive Abschnitte davon codieren, sowie Nukleinsäurefragmente, die zur Verwendung als Hybridisierungssonden oder Primer zur Identifizierung oder Amplifizierung von PTS-codierenden Nukleinsäuren (z.B. PTS-DNA) hinreichen. Der Begriff "Nukleinsäuremolekül" soll DNA-Moleküle (z.B. cDNA oder genomische DNA) und RNA-Moleküle (z.B. mRNA) sowie DNA- oder RNA-Analoga, die mittels Nukleotidanaloga erzeugt werden, umfassen. Dieser Begriff umfaßt zudem die am 3'- und am 5'-Ende des codierenden Genbereichs gelegene untranslatierte Sequenz: mindestens etwa 100 Nukleotide der Sequenz stromaufwärts des 5'-Endes des codierenden Bereichs und mindestens etwa 20 Nukleotide der Sequenz stromabwärts des 3'-Endes des codierenden Genbereichs. Das Nukleinsäuremolekül kann einzelsträngig oder doppelsträngig sein, ist aber vorzugsweise eine doppelsträngige DNA. Ein "isoliertes" Nukleinsäuremolekül wird aus anderen Nukleinsäuremolekülen abgetrennt, die in der natürlichen Quelle der Nukleinsäure zugegen sind. Eine "isolierte" Nukleinsäure hat vorzugsweise keine Sequenzen, die die Nukleinsäure in der genomischen DNA des Organismus, aus dem die Nukleinsäure stammt, natürlicherweise flankieren (bspw. Sequenzen, die sich am 5'- bzw. 3'-Ende der Nukleinsäure befinden). In verschiedenen Ausführungsformen kann bspw. das isolierte PTS-Nukleinsäuremolekül weniger als etwa 5 kb, 4 kb, 3 kb, 2 kb, 1 kb, 0,5 kb oder 0,1 kb der Nukleotidsequenzen, die natürlicherweise das Nukleinsäuremolekül in der genomischen DNA der Zelle, aus der die Nukleinsäure stammt (bspw. eine *C. glutamicum-*Zelle*)* flankieren. Ein "isoliertes" Nukleinsäuremolekül, wie ein cDNA-Molekül kann überdies im wesentlichen frei von einem anderen zellulären Material oder Kulturmedium sein, wenn es durch rekombinante Techniken hergestellt wird, oder frei von chemischen Vorstufen oder anderen Chemikalien sein, wenn es chemisch synthetisiert wird.

Ein erfindungsgemäßes Nukleinsäuremolekül, bspw. eine Nukleinsäuremolekül mit einer Nukleotidsequenz aus Anhang A oder ein Abschnitt davon, kann mittels molekularbiologischer Standard-Techniken und der hier bereitgestellten Sequenzinformation hergestellt werden. Bspw. kann eine *C. glutamicum-*PTS-cDNA aus einer *C. glutamicum-*Bank isoliert werden, indem eine vollständige Sequenz aus Anhang A oder ein Abschnitt davon als Hybridisierungssonde und Standard-Hybridisierungstechniken (wie bspw. beschrieben in Sambrook, J., Fritsch, E.F. und Maniatis, T. Molecular Cloning: A Laboratory Manual. 2. Aufl. Cold Spring Harbor Laboratory, Cold Spring Harbor Laboratory Press, Cold Spring Harbor, NY, 1989) verwendet werden. Überdies läßt sich ein Nukleinsäuremolekül, umfassend eine vollständige Sequenz aus Anhang A oder ein Abschnitt davon, durch Polymerasekettenreaktion isolieren, wobei die Oligonukleotidprimer, die auf der Basis dieser Sequenz erstellt wurden, verwendet werden (z.B. kann ein Nukleinsäuremolekül, umfassend eine vollständige Sequenz aus Anhang A oder einen Abschnitt davon, durch Polymerasekettenreaktion isoliert werden, indem Oligonukleotidprimer verwendet werden, die auf der Basis dieser gleichen Sequenz aus Anhang A erstellt worden sind). Bspw. läßt sich mRNA aus normalen Endothelzellen isolieren (bspw. durch das Guanidiniumthiocyanat-Extraktionsverfahren von Chirgwin et al. (1979) Biochemistry 18: 5294-5299) und die cDNA kann mittels reverser Transkriptase (bspw. Moloney-MLV-Reverse-Transkriptase, erhältlich bei Gibco/BRL, Bethesda, MD, oder AMV-Reverse-Transkriptase, erhältlich von Seikagaku America, Inc., St. Petersburg, FL) hergestellt werden. Synthetische Oligonukleotidprimer für die Amplifizierung via Polymerasekettereaktion lassen sich auf der Basis einer der in Anhang A gezeigten Nukleotidsequenzen erstellen. Eine erfindungsgemäße Nukleinsäure kann mittels cDNA oder alternativ genomischer DNA als Matrize und geeigneten Oligonukleotidprimern gemäß PCR-Standard-Amplifikationstechniken amplifiziert werden. Die so amplifizierte Nukleinsäure kann in einen geeigneten Vektor kloniert werden und durch DNA-Sequenzanalyse charakterisiert werden. Oligonukleotide, die einer PTS-Nukleotidsequenz entsprechen, können durch Standard-Syntheseverfahren, bspw. mit einem automatischen DNA-Synthesegerät, hergestellt werden.

Bei einer bevorzugten Ausführungsform umfaßt ein erfindungsgemäßes isoliertes Nukleinsäuremolekül eine der in Anhang A aufgeführten Nukleotidsequenzen.

Bei einer weiteren bevorzugten Ausführungsform umfaßt ein erfindungsgemäßes isoliertes Nukleinsäuremolekül ein zu einer der in Anhang A gezeigten Nukleotidsequenzen komplementäres Nukleinsäuremolekül oder einen Abschnitt davon, wobei es sich um ein Nukleinsäuremolekül handelt, das zu einer der in Anhang A gezeigten Nukleotidsequenzen hinreichend komplementär ist, daß es mit einer der in Anhang A angegebenen Sequenzen hybridisieren kann, wodurch ein stabiler Duplex entsteht.

Bei einer Ausführungsform codiert das erfindungsgemäße Nukleinsäuremolekül ein Protein oder einen Abschnitt davon, der eine Aminosäuresequenz umfaßt, die hinreichend homolog zu einer Aminosäuresequenz von Anhang B ist, daß das Protein oder ein Abschnitt davon weiterhin am Transport von energiereichen Kohlenstoffmolekülen (wie Glucose) in *C. glutamicum* und auch an einem oder mehreren intrazellulären Signaltransduktionswegen beteiligt sein kann. Wie hier verwendet, betrifft der Begriff "hinreichend homolog" Proteine oder Abschnitte davon, deren Aminosäuresequenzen eine minimale Anzahl identischer oder äquivalenter Aminosäurereste (bspw. ein Aminosäurerest mit einer ähnlichen Seitenkette wie ein Aminosäurerest in einer der Sequenzen von Anhang B) zu einer Aminosäuresequenz aus Anhang B aufweisen, so daß das Protein oder ein Abschnitt davon energiereiche Kohlenstoffmoleküle, wie Glucose, in *C. glutamicum* transportieren kann und zudem an der intrazellulären Signaltransduktion in diesem Mikroorganismus beteiligt sein kann. Proteinbestandteile dieser Stoffwechselwege transportieren, wie hier beschrieben, energiereiche kohlenstoffhaltige Moleküle wie Glucose in *C. glutamicum* und können auch an der intrazellulären Signaltransduktion in diesem Mikroorganismus beteiligt sein. Beispiele dieser Aktivitäten sind ebenfalls hier beschrieben. Somit betrifft die "Funktion eines PTS-Proteins" das vollständige Funktionieren und/oder die Regulation von einem oder mehreren Zuckertransport-Wegen auf Phosphoenolpyruvat-Basis. In Tabelle 1 sind Beispiele der PTS-Proteinaktivitäten angegeben.

Abschnitte von Proteinen, die von den erfindungsgemäßen PTS-Nukleinsäuremolekülen codiert werden, sind vorzugsweise biologisch aktive Abschnitte von einem der PTS-Proteine. Der Begriff "biologisch aktiver Abschnitt eines PTS-Proteins", wie er hier verwendet wird, soll einen Abschnitt, bspw. eine Domäne oder ein Motiv, eines PTS-Proteins umfassen, die/das energiereiche kohlenstoffhaltige Moleküle, wie Glucose, in *C. glutamicum* transportieren kann, oder an der intrazellulären Signaltransduktion in diesem Mikroorganismus beteiligt sein kann, oder eine in Tabelle 1 angegebene Aktivität aufweist. Zur Bestimmung, ob ein PTS-Protein oder ein biologisch aktiver Abschnitt davon am Transport von energiereichen kohlenstoffhaltigen Molekülen, wie Glucose, in *C. glutamicum* oder an der intrazellulären Signaltransduktion in diesem Mikroorganismus beteiligt sein kann, kann ein Test der enzymatischen Aktivität durchgeführt werden. Diese Testverfahren, wie eingehend beschrieben in Beispiel 8 des Beispielteils, sind dem Fachmann geläufig.

Zusätzlich zu natürlich vorkommenden Varianten der PTS-Sequenz, die in der Population existieren können, ist der Fachmann sich ebenfalls dessen bewußt, daß Änderungen durch Mutation in eine Nukleotidsequenz von Anhang A eingebracht werden können, was zur Änderung der Aminosäuresequenz des codierten PTS-Proteins führt, ohne daß die Funktionsfähigkeit des PTS-Proteins beeinträchtigt wird. Bspw. lassen sich Nukleotidsusbtitutionen, die an "nicht-essentiellen" Aminosäureresten zu Aminosäuresubstitutionen führen, in einer Sequenz von Anhang A herstellen. Ein "nicht-essentieller" Aminosäurerest läßt sich in einer Wildtypsequenz von einem der PTS-Proteine (Anhang B) verändern, ohne daß die Aktivität des PTS-Proteins verändert wird, wohingegen ein "essentieller" Aminosäurerest für die PTS-Proteinaktivität erforderlich ist. Andere Aminosäurereste jedoch (bspw. nicht-konservierte oder lediglich semikonservierte Aminosäurereste in der Domäne mit PTS-Aktivität) können für die Aktivität nicht essentiell sein und lassen sich somit wahrscheinlich verändern, ohne daß die PTS-Aktivität verändert wird.

Ein isoliertes Nukleinsäuremolekül, das ein PTS-Protein codiert, das zu einer Proteinsequenz aus Anhang B homolog ist, kann durch Einbringen von einer oder mehreren Nukleotidsubstitutionen, -additionen oder -deletionen in eine Nukleotidsequenz aus Anhang A erzeugt werden, so daß eine oder mehrere Aminosäuresubstitutionen, -additionen oder -deletionen in das codierte Protein eingebracht werden. Die Mutationen können in eine der Sequenzen aus Anhang A durch Standard-Techniken eingebracht werden, wie stellengerichtete Mutagenese und PCR-vermittelte Mutagenese. Vorzugsweise werden konservative Aminosäuresubstitutionen an einer oder mehreren der vorhergesagten nicht-essentiellen Aminosäureresten eingeführt. Bei einer "konservativen Aminosäuresubstitution" wird der Aminosäurerest durch einen Aminosäurerest mit einer ähnlichen Seitenkette ausgetauscht. Im Fachgebiet sind Familien von Aminosäureresten mit ähnlichen Seitenketten definiert worden. Diese Familien umfassen Aminosäuren mit basischen Seitenketten (z.B. Lysin, Arginin, Histidin), sauren Seitenketten (z.B. Asparaginsäure, Glutaminsäure), ungeladenen polaren Seitenketten (z.B. Glycin, Asparagin, Glutamin, Serin, Threonin, Tyrosin, Cystein), nicht-polaren Seitenketten, (bspw. Alanin, Valin, Leucin, Isoleucin, Prolin, Phenylalanin, Methionin, Tryptophan), betaverzweigten Seitenketten (z.B. Threonin, Valin, Isoleucin) und aromatischen Seitenketten (z.B. Tyrosin, Phenylalanin, Tryptophan, Histidin). Ein vorhergesagter nicht-essentieller Aminosäurerest in einem PTS-Protein wird somit vorzugsweise durch einen anderen Aminosäurerest der gleichen Seitenkettenfamilie ausgetauscht. In einer weiteren Ausführungsform können die Mutationen alternativ zufallsgemäß über die gesamte oder einen Teil der PTS-codierenden Sequenz eingebracht werden, bspw. durch Sättigungsmutagenese, und die resultierenden Mutanten können auf die hier beschriebene PTS-Aktivität untersucht werden, um Mutanten zu identifizieren, die eine PTS-Aktivität beibehalten. Nach der Mutagenese von einer der Sequenzen aus Anhang A kann das codierte Protein rekombinant exprimiert werden, und die Aktivität des Proteins kann bspw. mit den hier beschriebenen Tests (siehe Beispiel 8 des Beispielteils) bestimmt werden.

### B. Rekombinante Expressionsvektoren und Wirtszellen

Ein weiterer Aspekt der Erfindung betrifft Vektoren, vorzugsweise Expressionsvektoren, die eine Nukleinsäure enthalten, die ein PTS-Protein (oder einen Abschnitt davon) codieren. Wie hier verwendet betrifft der Begriff "Vektor" ein Nukleinsäuremolekül, das eine andere Nukleinsäure transportieren kann, an welche es gebunden ist. Ein Vektortyp ist ein "Plasmid", was für eine zirkuläre doppelsträngige DNA-Schleife steht, in die zusätzlichen DNA-Segmente ligiert werden können. Ein weiterer Vektortyp ist ein viraler Vektor, wobei zusätzliche DNA-Segmente in das virale Genom ligiert werden können. Bestimmte Vektoren können in einer Wirtszelle, in die sie eingebracht worden sind, autonom replizieren (bspw. Bakterienvektoren, mit bakteriellem Replikationsursprung und episomale Säugetiervektoren). Andere Vektoren (z.B. nichtepisomale Säugetiervektoren) werden in das Genom einer Wirtszelle beim Einbringen in die Wirtszelle integriert und dadurch zusammen mit dem Wirtsgenom repliziert. Zudem können bestimmte Vektoren die Expression von Genen, mit denen sie funktionsfähig verbunden sind, steuern. Diese Vektoren werden als "Expressionsvektoren" bezeichnet. Gewöhnlich haben die Expressionsvektoren, die bei DNA-Rekombinationstechniken verwendet werden, die Form von Plasmiden. In der vorliegenden Beschreibung können "Plasmid" und "Vektor" austauschbar verwendet werden, da das Plasmid die am häufigsten verwendete Vektorform ist. Die Erfindung soll diese anderen Expressionsvektorformen, wie virale Vektoren (bspw. replikationsdefiziente Retroviren, Adenoviren und adenoverwandte Viren), die ähnliche Funktionen ausüben, umfassen.

Der erfindungsgemäße rekombinante Expressionsvektor umfaßt eine erfindungsgemäße Nukleinsäure in einer Form, die sich zur Expression der Nukleinsäure in einer Wirtszelle eignet, was bedeutet, daß die rekombinanten Expressionsvektoren eine oder mehrere regulatorische Sequenzen, ausgewählt auf der Basis der zur Expression zu verwendenden Wirtszellen, die mit der zu exprimierenden Nukleinsäuresequenz funktionsfähig verbunden ist, umfaßt. In einem rekombinanten Exprtessionsvektor bedeutet "funktionsfähig verbunden", daß die Nukleotidsequenz von Interesse derart an die regulatorische(n) Sequenz(en) gebunden ist, daß die Expression der Nukleotidsequenz möglich ist (bspw. in einem In-vitro-Transkriptions-/Translationssystem oder in einer Wirtszelle, wenn der Vektor in die Wirtszelle eingebracht ist). Der Begriff "regulatorische Sequenz" soll Promotoren, Enhancer und andere Expressionskontrollelemente (bspw. Polyadenylierungssignale) umfassen. Diese regulatorischen Sequenzen sind bspw beschrieben in Goeddel: Gene Expression Technology: Methods in Enzymology 185, Academic Press, San Diego, CA (1990). Regulatorische Sequenzen umfassen solche, die die konstitutive Expression einer Nukleotidsequenz in vielen Wirtszelltypen steuern, und solche, die die direkte Expression der Nukleotidsequenz nur in bestimmten Wirtszellen steuern. Der Fachmann ist sich dessen bewußt, daß die Gestaltung eines Expressionsvektors von Faktoren abhängen kann, wie der Wahl der zu transformierenden Wirtszelle, dem Ausmaß der Expression des gewünschten Proteins usw. Die erfindungsgemäßen Expressionsvektoren können in die Wirtszellen eingebracht werden, so daß dadurch Proteine oder Peptide, einschließlich Fusionsproteinen oder -peptiden, die von den Nukleinsäuren, wie hier beschrieben, codiert werden, hergestellt werden (bspw. PTS-Proteine, mutante Formen von PTS-Proteinen, Fusionsproteine, usw.).

Die erfindungsgemäßen rekombinanten Expressionsvektoren können zur Expression von PTS-Proteinen in prokaryotischen oder eukaryotischen Zellen ausgestaltet sein. Bspw. können PTS-Gene in bakteriellen Zellen, wie *C. glutamicum,* Insektenzellen (mit Baculovirus-Expressionsvektoren), Hefe- und anderen Pilzzellen (siehe Romanos, M.A. et al. (1992) "Foreign gene expression in yeast: a review", Yeast 8: 423-488; van den Hondel, C.A.M.J.J. et al. (1991) "Heterologous gene expression in filamentous fungi" in: More Gene Manipulations in Fungi, J.W. Bennet & L.L. Lasure, Hrsg., S. 396-428: Academic Press: San Diego; und van den Hondel, C.A.M.J.J. & Punt, P.J. (1991) "Gene transfer systems and vector development for filamentous fungi. in: Applied Molecular Genetics of Fungi, Peberdy, J.F. et al., Hrsg, S. 1-28, Cambridge University Press: Cambridge), Algen- und vielzelligen Pflanzenzellen (siehe Schmidt, R. und Willmitzer, L. (1988) "High efficiency Agrobacterium tumefaciens-mediated transformation of Arabidopsis thaliana leaf and cotyledon explants" Plant Cell Rep.: 583-586) oder Säugetierzellen exprimiert werden. Geeignete Wirtszellen werden weiter erörtert in Goeddel, Gene Expression Technology: Methods in Enzymology 185, Academic Press, San Diego, CA (1990). Der rekombinante Expressionsvektor kann alternativ, bspw. mit T7-Promotorregulatorischen Sequenzen und T7-Polymerase, in vitro transkribiert und translatiert werden.

Die Expression von Proteinen in Prokaryonten erfolgt meist mit Vektoren, die konstitutive oder induzierbare Promotoren enthalten, die die Expression von Fusions- oder Nicht-Fusionsproteinen steuern. Fusionsvektoren steuern eine Reihe von Aminosäuren zu einem darin codierten Protein, gewöhnlich am Aminoterminus des rekombinanten Proteins, bei. Diese Fusionsvektoren haben gewöhnlich drei Aufgaben: 1) die Verstärkung der Expression von rekombinantem Protein; 2) die Erhöhung der Löslichkeit des rekombinanten Proteins; und 3) die Unterstützung der Reinigung des rekombinanten Proteins durch Wirkung als Ligand bei der Affinitätsreinigung. Bei Fusions-Expressionsvektoren wird oft eine proteolytische Spaltstelle an der Verbindungsstelle der Fusionseinheit und des rekombinanten Proteins eingebracht, so daß die Trennung des rekombinanten Proteins von der Fusionseinheit nach der Reinigung des Fusionsproteins möglich ist. Diese Enzyme und ihre entsprechenden Erkennungssequenzen umfassen Faktor Xa, Thrombin und Enterokinase.

Übliche Fusionsexpressionsvektoren umfassen pGEX (Pharmacia Biotech Inc; Smith, D.B. und Johnson, K.S. (1988) Gene 67: 31-40), pMAL (New England Biolabs, Beverly, MA) und pRIT 5 (Pharmacia, Piscataway, NJ), bei denen Glutathion-S-Transferase (GST), Maltose E-bindendes Protein bzw. Protein A an das rekombinante Zielprotein fusioniert wird. Bei einer Ausführungsform ist die codierende Sequenz des PTS-Proteins in einen pGEX-Expressionsvektor kloniert, so daß ein Vektor erzeugt wird, der ein Fusionsprotein codiert, umfassend vom N-Terminus zum C-Terminus, GST - Thrombin-Spaltstelle - X-Protein. Das Fusionsprotein kann durch Affinitätschromatographie mittels Glutathion-Agarose-Harz gereinigt werden. Das rekombinante PTS-Protein, das nicht mit GST fusioniert ist, kann durch Spaltung des Fusionsproteins mit Thrombin gewonnen werden.

Beispiele geeigneter induzierbarer Nicht-Fusions-Expressionsvektoren aus E. coli umfassen pTrc (Amann et al., (1988) Gene 69: 301 - 315) und pET 11d (Studier et al. Gene Expression Technology: Methods in Enzymology 185, Academic Press, San Diego, Kalifornien (1990) 60-89). Die Zielgenexpression aus dem pTrc-Vektor beruht auf der Transkription durch Wirts-RNA-Polymerase von einem Hybrid-trp-lac-Fusionspromotor. Die Zielgenexpression aus dem pET11d-Vektor beruht auf der Transkription von einem T7-gn10-lac-Fusions-Promotor, die von einer coexprimierten viralen RNA-Polymerase (T7 gn1) vermittelt wird. Diese virale Polymerase wird von den Wirtsstämmen BL 21 (DE3) oder HMS174 (DE3) von einem residenten λ-Prophagen geliefert, der ein T7 gnl-Gen unter der Transkriptionskontrolle des lacUV 5-Promotors birgt.

Eine Strategie zur Maximierung der Expression des rekombinanten Proteins ist die Expression des Proteins in einem Wirtsbakterium, dessen Fähigkeit zur proteolytischen Spaltung des rekombinanten Proteins gestört ist (Gottesman, S. Gene Expression Technology: Methods in Enzymology 185, Academic Press, San Diego, Kalifornien (1990) 119-128). Eine weitere Strategie ist die Veränderung der Nukleinsäuresequenz der in einen Expressionsvektor zu inserierenden Nukleinsäure, so daß die einzelnen Codons für jede Aminosäure diejenigen sind, die vorzugsweise in einem zur Expression ausgewählten Bakterium, wie *C. glutamicum,* verwendet werden (Wada et al. (1992) Nucleic Acids Res. 20: 2111 - 2118). Diese Veränderung der erfindungsgemäßen Nukleinsäuresequenzen erfolgt durch Standard-DNA-Synthesetechniken.

Bei einer weiteren Ausführungsform ist der PTS-Proteinexpressionsvektor ein Hefe-Expressionsvektor. Beispiele für Vektoren zur Expression in der Hefe *S. cerevisiae* umfassen pYepSeal (Baldari et al., (1987) Embo J. 6: 229-234), pMFa (Kurjan und Herskowitz (1982) Cell 30: 933-943), pJRY88 (Schultz et al. (1987) Gene 54: 113 - 123) sowie pYES2 (Invitrogen Corporation, San Diego, CA). Vektoren und Verfahren zur Konstruktion von Vektoren, die sich zur Verwendung in anderen Pilzen, wie filamentösen Pilzen, eignen, umfassen diejenigen, die eingehend beschrieben sind in: van den Hondel, C.A.M.J.J. & Punt, P.J. (1991) "Gene transfer systems and vector development for filamentous fungi, in: Applied Molecular Genetics of fungi, J.F. Peberdy et al., Hrsg., S. 1-28, Cambridge University Press: Cambridge.

Alternativ können die erfindungsgemäßen PTS-Proteine in Insektenzellen unter Verwendung von Baculovirus-Expressionsvektoren exprimiert werden. Baculovirus-Vektoren, die zur Expression von Proteinen in gezüchteten Insektenzellen (bspw. Sf9-Zellen) verfügbar sind, umfassen die pAc-Reihe (Smith et al., (1983) Mol. Cell Biol.. 3: 2156-2165) und die pVL-Reihe (Lucklow und Summers (1989) Virology 170: 31-39).

In einer weiteren Ausführungsform können die erfindungsgemäßen PTS-Proteine in einzelligen Pflanzenzellen (wie Algen) oder in Pflanzenzellen höherer Pflanzen (bspw. Spermatophyten, wie Feldfrüchte) exprimiert werden. Beispiele für Pflanzen-Expressionsvektoren umfassen solche, die eingehend beschrieben sind in: Bekker, D., Kemper, E., Schell, J. und Masterson, R. (1992) "New plant binary vectors with selectable markers located proximal to the left border", Plant Mol. Biol. 20: 1195-1197; und Bevan, M.W. (1984) "Binary Agrobacterium vectors for plant transformation", Nucl. Acids Res. 12: 8711-8721.

In einer weiteren Ausführungsform wird eine erfindungsgemäße Nukleinsäure in Säugetierzellen mit einem Säugetier-Expressionsvektor exprimiert.. Beispiele für Säugetier-Expressionsvektoren umfassen pCDM8 (Seed, B. (1987) Nature 329:840) und pMT2PC (Kaufman et al. (1987) EMBO J. 6: 187-195). Bei der Verwendung in Säugetierzellen werden die Kontrollfunktionen des Expressionsvektors oft von viralen regulatorischen Elementen bereitgestellt. Gemeinhin verwendete Promotoren stammen bspw. aus Polyoma, Adenovirus2, Cytomegalievirus und Simian Virus 40. Für weitere geeignete Expressionssysteme für prokaryotische und eukaryotische Zellen, siehe die Kapitel 16 und 17 aus Sambrook, J., Fritsch, E.F. und Maniatis, T., Molecular cloning: A Laboratory Manual, 2. Auflage, Cold Spring Harbor Laboratory, Cold Spring Harbor Laboratory Press, Cold Spring Harbor, NY, 1989.

Bei einer weiteren Ausführungsform kann der rekombinante Säugetier-Expressionsvektor die Expression der Nukleinsäure vorzugsweise in einem bestimmten Zelltyp bewirken (bspw. werden gewebespezifische regulatorische Elemente zur Expression der Nukleinsäure verwendet). Gewebespezifische regulatorische Elemente sind im Fachgebiet bekannt. Nicht-einschränkende Beispiele für geeignete gewebespezifische Promotoren umfassen den Albuminpromotor (leberspezifisch; Pinkert et al. (1987) Genes Dev. 1: 268-277), lymphoid-spezifische Promotoren (Calame und Eaton (1988) Adv. Immunol. 43: 235-275), insbesondere Promotoren von T-Zellrezeptoren (Winoto und Baltimore (1989) EMBO J. 8: 729-733) und Immunglobulinen (Banerji et al. (1983) Cell 33: 729-740; Queen und Baltimore (1983) Cell 33: 741-748), neuronspezifische Promotoren (bspw. Neurofilament-Promotor; Byrne und Ruddle (1989) PNAS 86: 5473-5477), pankreasspezifische Promotoren (Edlund et al., (1985) Science 230: 912-916) und milchdrüsenspezifische Promotoren (bspw. Milchserum-Promotor; US-Patent Nr. 4 873 316 und europäische Patentanmeldungsveröffentlichung Nr. 264 166). Entwicklungsregulierte Promoren sind ebenfalls umfaßt, bspw. die Maus-hox-Promotoren (Kessel und Gruss (1990) Science 249: 374-379) und der α-Fetoprotein-Promotor (Campes und Tilghman (1989) Genes Dev. 3: 537-546).

Die Erfindung stellt zudem einen rekombinanten Expressionsvektor bereit, umfassend ein erfindungsgemäßes DNA Molekül, das in Antisense-Richtung in den Expressionsvektor kloniert ist. Dies bedeutet, daß das DNA-Molekül derart mit einer regulatorischen Sequenz funktionsfähig verbunden ist, daß die Expression (durch Transkription des DNA-.Moleküls) eines RNA-Moleküls, das zur PTS-mRNA antisense ist, möglich ist. Es können regulatorische Sequenzen ausgewählt werden, die funktionsfähig an eine in Antisense-Richtung klonierte Nukleinsäure gebunden sind und die die kontinuierliche Expression des Antisense-RNA-Moleküls in einer Vielzahl von Zelltypen steuern, bspw. können virale Promotoren und/oder Enhancer oder regulatorische Sequenzen ausgewählt werden, die die konstitutive, gewebespezifische oder zelltypspezifische Expression von Antisense-RNA steuern. Der Antisense-Expressionsvektor kann in Form eines rekombinanten Plasmids, Phagemids oder attenuierten Virus vorliegen, in dem Antisense-Nukleinsäuren unter der Kontrolle eines hochwirksamen regulatorischen Bereichs produziert werden, dessen Aktivität durch den Zelltyp bestimmt wird, in den der Vektor eingebracht wird. Für eine Diskussion der Regulation der Genexpression mittels Antisense-Genen, siehe Weintraub, H. et al., Antisense-RNA as a molecular tool for genetic analysis, Reviews - Trends in Genetics, Bd. 1(1) 1986.

Ein weiterer Aspekt der Erfindung betrifft die Wirtszellen, in die ein erfindungsgemäßer rekombinanter Expressionsvektor eingebracht worden ist. Die Begriffe "Wirtszelle" und "rekombinante Wirtszelle" werden hier untereinander austauschbar verwendet. Es ist selbstverständlich, daß diese Begriffe nicht nur eine bestimmte Zielzelle, sondern auch die Nachkommen oder potentiellen Nachkommen dieser Zelle betreffen. Da in aufeinanderfolgenden Generationen aufgrund von Mutation oder Umwelteinflüssen bestimmte Modifikationen auftreten können, sind diese Nachkommen nicht unbedingt mit der Parentalzelle identisch, sind jedoch im Umfang des Begriffs, wie er hier verwendet wird, noch umfaßt.

Eine Wirtszelle kann eine prokaryotische oder eukaryotische Zelle sein. Bspw. kann ein PTS-Protein in Bakterienzellen, wie *C. glutamicum,* Insektenzellen, Hefe- oder Säugetierzellen (wie Ovarzellen des chinesischen Hamsters (CHO) oder COS-Zellen) exprimiert werden. Andere geeignete Wirtszellen sind dem Fachmann geläufig. Mikroorganismen, die mit *Corynebacterium glutamicum* verwandt sind und sich geeignet als Wirtszellen für die erfindungsgemäßen Nukleinsäure- und Proteinmoleküle verwenden lassen, sind in Tabelle 3 aufgeführt.

Durch herkömmliche Transformations- oder Transfektionsverfahren läßt sich Vektor-DNA in prokaryotische oder eukaryotische Zellen einbringen. Die Begriffe "Transformation" und "Transfektion", wie sie hier verwendet werden, sollen eine Vielzahl von im Stand der Technik bekannten verfahren zum Einbringen fremder Nukleinsäure (bspw. DNA) in eine Wirtszelle umfassen, einschließlich Calciumphosphat- oder Calciumchlorid-Copräzipitation, DEAE-Dextran-vermittelte Transfektion, Lipofektion oder Elektroporation. Geeignete Verfahren zur Transformation oder Transfektion von Wirtszellen lassen sich nachlesen in Sambrook et al. (Molecular Cloning: A Laboratory Manual. 2. Aufl. Cold Spring Harbor Laboratory, Cold Spring Harbor Laboratory Press, Cold Spring Harbor, NY, 1989) und anderen Labor-Handbüchern.

Für die stabile Transfektion von Säugetierzellen ist bekannt, daß je nach verwendetem Expressionsvektor und verwendeter Transfektionstechnik nur ein kleiner Teil der Zellen die fremde DNA in ihr Genom integriert. Zur Identifizierung und Selektion dieser Integranten wird gewöhnlich ein Gen, das einen selektierbaren Marker (z.B. Resistenz gegen Antibiotika) codiert, zusammen mit dem Gen von Interesse in die Wirtszellen eingebracht. Bevorzugte selektierbare Marker umfassen solche, die die Resistenz gegen Medikamente, wie G418, Hygromycin und Methotrexat, verleihen. Eine Nukleinsäure, die einen selektierbaren Marker codiert, kann in eine Wirtszelle auf dem gleichen Vektor eingebracht werden, wie derjenige, der ein PTS-Protein codiert, oder kann auf einem gesonderten Vektor eingebracht werden. Zellen, die mit der eingebrachten Nukleinsäure stabil transfiziert worden sind, können durch Medikamentenselektion identifiziert werden (z.B. Zellen, die den selektierbaren Marker integriert haben, überleben, wohingegen die anderen Zellen sterben).

Zur Erzeugung eines homolog rekombinierten Mikroorganismus wird ein Vektor hergestellt, der zumindest einen Abschnitt eines PTS-Gens enthält, in den eine Deletion, Addition oder Substitution eingebracht worden ist, um das PTS-Gen zu verändern, bspw. funktionell zu disrumpieren. Dieses PTS-Gen ist vorzugsweise ein *Corynebacterium glutamicum-*PTS*-*Gen, jedoch kann ein Homologon von einem verwandten Bakterium oder sogar von einer Säugetier-, Hefe- oder Insektenquelle verwendet werden. Bei einer bevorzugten Ausführungsform ist der Vektor derart ausgestaltet, daß das endogene PTS-Gen bei homologer Rekombination funktionell disrumpiert ist (d.h. nicht länger ein funktionelles Protein codiert, ebenfalls bezeichnet als "Knockout"-Vektor). Der Vektor kann alternativ derart ausgestaltet sein, daß das endogene PTS-Gen bei homologer Rekombination mutiert oder anderweitig verändert ist, jedoch noch das funktionelle Protein codiert (z.B. kann der stromaufwärts gelegene regulatorische Bereich derart verändert sein, daß dadurch die Expression des endogenen PTS-Proteins verändert wird.). Der veränderte Abschnitt des PTS-Gens ist im homologen Rekombinationsvektor an seinem 5'- und 3'-Ende von zusätzlicher Nukleinsäure des PTS-Gens flankiert, die eine homologe Rekombination zwischen dem exogenen PTS-Gen, das von dem Vektor getragen wird, und einem endogenen PTS-Gen in einem Mikroorganismus, ermöglicht. Die zusätzliche flankierende PTS-Nukleinsäure ist für eine erfolgreiche homologe Rekombination mit dem endogenen Gen hinreichend lang. Gewöhnlich enthält der Vektor mehrere Kilobasen flankierende DNA (sowohl am 5'- als auch am 3'-Ende) (siehe z.B. Thomas, K.R. und Capecchi, M.R. (1987) Cell 51: 503 für eine Beschreibung von homologen Rekombinationsvektoren). Der Vektor wird in einen Mikroorganismus (z.B. durch Elektroporation) eingebracht, und Zellen, in denen das eingebrachte PTS-Gen mit dem endogenen PTS-Gen homolog rekombiniert ist, werden unter Verwendung im Fachgebiet bekannter Verfahren selektiert.

Bei einer anderen Ausführungsform können rekombinante Mikroorganismen produziert werden, die ausgewählte Systeme enthalten, die eine regulierte Expression des eingebrachten Gens ermöglichen. Der Einschluß eines PTS-Gens in einem Vektor unter der Kontrolle des Lac-Operons ermöglicht z.B. die Expression des PTS-Gens nur in Gegenwart von IPTG. Diese regulatorischen Systeme sind im Fachgebiet bekannt.

Eine erfindungsgemäße Wirtszelle, wie eine prokaryotische oder eukaryotische Wirtszelle in Kultur, kann zur Produktion (d.h. Expression) eines PTS-Proteins verwendet werden. Die Erfindung stellt zudem Verfahren zur Produktion von PTS-Proteinen unter Verwendung der erfindungsgemäßen Wirtszellen bereit. Bei einer Ausführungsform umfaßt das Verfahren die Anzucht der erfindungsgemäßen Wirtszelle (in die ein rekombinanter Expressionsvektor, der ein PTS-Protein codiert, eingebracht worden ist, oder in deren Genom ein Gen eingebracht worden ist, das ein Wildtyp- oder verändertes PTS-Protein codiert) in einem geeigneten Medium, bis das PTS-Protein produziert worden ist. Das Verfahren umfaßt in einer weiteren Ausführungsform das Isolieren der PTS-Proteine aus dem Medium oder der Wirtszelle.

### C. Erfindungsgemäße Verwendungen und Verfahren

Die hier beschriebenen Nukleinsäuremoleküle, Proteine, Proteinhomologa, Fusionsproteine, Primer, Vektoren und Wirtszellen können in einem oder mehreren nachstehenden Verfahren verwendet werden: Identifikation von *C. glutamicum* und verwandten Organismen, Kartierung von Genomen von Organismen, die mit *C. glutamicum* verwandt sind, Identifikation und Lokalisation von *C. glutamicum-Se*quenzen von Interesse, Evolutionsstudien, Bestimmung von PTS-Proteinbereichen, die für die Funktion notwendig sind, Modulation der Aktivität eines PTS-Proteins; Modulation der Aktivität eines PTS-Wegs; und Modulation der zellulären Produktion einer gewünschten Verbindung, wie einer Feinchemikalie. Die erfindungsgemäßen PTS-Nukleinsäuremoleküle haben eine Vielzahl von Verwendungen. Sie können zunächst zur Identifikation eines Organismus als *Corynebacterium glutamicum* oder naher Verwandten davon verwendet werden. Sie können zudem zur Identifikation von *C. glutamicum* oder eines Verwandten davon in einer Mischpopulation von Mikroorganismen verwendet werden. Die Erfindung stellt die Nukleinsäuresequenzen einer Reihe von *C. glutamicum-*Genen bereit. Durch Sondieren der extrahierten genomischen DNA einer Kultur einer einheitlichen oder gemischten Population von Mikroorganismen unter stringenten Bedingungen mit einer Sonde, die einen Bereich eines *C. glutamicum*-Gens umfaßt, das für diesen Organismus einzigartig ist, kann man bestimmen, ob dieser Organismus zugegen ist. *Corynebacterium glutamicum* selbst ist zwar nicht pathogen, jedoch ist es mit pathogenen Arten, wie *Corynebaaterium diptheriae,* verwandt. Der Nachweis eines solchen Organismus ist von signifikanter klinischer Bedeutung.

Die erfindungsgemäßen Nukleinsäure- und Proteinmoleküle können als Marker für spezifische Bereiche des Genoms dienen. Dies ist nicht nur beim Kartieren des Genoms, sondern auch für funktionelle Studien von *C*. *glutamicum*-Proteinen nützlich. Zur Identifikation des Genombereichs, an den ein bestimmtes *C. glutamicum-*DNA-bindendes Protein bindet, kann das *C. glutamicum-Genom* bspw. gespalten werden, und die Fragmente mit dem DNA-bindenden Protein inkubiert werden. Diejenigen, die das Protein binden, können zusätzlich mit den erfindungsgemäßen Nukleinsäuremolekülen, vorzugsweise mit leicht nachweisbaren Markierungen, sondiert werden; die Bindung eines solchen Nukleinsäuremoleküls an das Genomfragment ermöglicht die Lokalisation des Fragmentes auf der genomischen Karte von *C. glutamicum,* und wenn dies mehrmals mit unterschiedlichen Enzymen durchgeführt wird, erleichtert es eine rasche Bestimmung der Nukleinsäuresequenz, an die das Protein bindet. Die erfindungsgemäßen Nukleinsäuremoleküle können zudem hinreichend homolog zu den Sequenzen verwandter Arten sein, so daß diese Nukleinsäuremoleküle als Marker für die Konstruktion einer genomischen Karte in verwandten Bakterien, wie *Brevibacterium lactofermentum,* dienen können.

Die erfindungsgemäßen PTS-Nukleinsäuremoleküle eignen sich ebenfalls für Evolutions- und Proteinstrukturuntersuchungen. Das Zukkeraufnahmesystem, an dem die erfindungsgemäßen Moleküle beteiligt sind, wird von vielen Bakterien ausgenutzt; durch Vergleich der Sequenzen der erfindungsgemäßen Nukleinsäuremoleküle mit solchen, die ähnliche Enzyme aus anderen Organismen codieren, kann der Evolutions-Verwandschaftsgrad der Organismen bestimmt werden. Entsprechend ermöglicht ein solcher Vergleich die Bestimmung, welche Sequenzbereiche konserviert sind und welche nicht, was bei der Bestimmung solcher Bereiche des Proteins hilfreich sein kann, die für die Enzymfunktion essentiell sind. Dieser Typ der Bestimmung ist für Proteintechnologie-Untersuchungen wertvoll und kann einen Hinweis darauf geben, welches Protein Mutagenese tolerieren kann, ohne die Funktion zu verlieren.

Die Manipulation der erfindungsgemäßen PTS-Nukleinsäuremoleküle kann die Produktion von PTS-Proteinen mit funktionellen Unterschieden zu den Wildtyp-PTS-Proteinen bewirken. Diese Proteine können hinsichtlich ihrer Effizienz oder Aktivität verbessert werden, können in größerer Anzahl als gewöhnlich in der Zelle zugegen sein, oder können hinsichtlich ihrer Effizienz oder Aktivität geschwächt sein.

Die erfindungsgemäßen PTS-Moleküle können derart modifiziert sein, daß die Ausbeute, Produktion und/oder Effizienz der Produktion von einer oder mehreren Feinchemikalien verbessert ist. Durch derartige Modifikation eines an der Glucoseaufnahme beteiligten PTS-Proteins, daß es optimale Aktivität aufweist, kann das Ausmaß der Glucoseaufnahme oder die Geschwindigkeit, mit der die Glucose in die Zelle befördert wird, vergrößert werden. Der Abbau von Glucose und anderen Zuckern innerhalb der Zelle stellt die Energie bereit, die zum Antrieb energetisch ungünstiger biochemischer Reaktionen, wie solchen, die an der Biosynthese von Feinchemikalien beteiligt sind, verwendet werden kann. Dieser Abbau stellt ebenfalls Zwischen- und Vorstufenmoleküle für die Biosynthese bestimmter Feinchemikalien, wie Aminosäuren, Vitamine und Cofactoren, bereit. Durch Erhöhung der Menge an intrazellulären energiereichen Kohlenstoffmolekülen durch Modifikation der erfindungsgemäßen PTS-Moleküle kann man daher die Energie, die zur Durchführung von Stoffwechselwegen, die zur Produktion von ein oder mehreren Feinchemikalien notwendig sind, verfügbar ist, und auch die intrazellulären Pools von Metaboliten, die für eine solche Produktion notwendig sind, vergrößern. Umgekehrt kann man durch Senken des Imports eines Zuckers, dessen Abbauprodukte eine Verbindung umfassen, die nur in Stoffwechselwegen verwendet werden, die mit Stoffwechselwegen zur Produktion einer gewünschten Feinchemikalie um Enzyme, Cofaktoren oder Zwischenprodukte konkurrieren, diesen Stoffwechselweg herunterregulieren und somit vielleicht die Produktion durch den gewünschten Biosyntheseweg vergrößern.

Die erfindungsgemäßen PTS-Moleküle können an einem oder mehreren intrazellulären Signaltransduktionswegen, die die Ausbeuten und/oder Produktionsrate von einer oder mehreren Feinchemikalien aus *C. glutamicum* beeinflussen, beteiligt sein. Bspw. werden Proteine, die für den Import von einem oder mehreren Zuckern aus dem extrazellulären Medium notwendig sind, (z.B. HPr, Enzym 1 oder ein Baustein eines Enzym-II-Komplexes) bei Anwesenheit einer hinreichenden Menge des Zuckers in der Zelle häufig posttranslational modifiziert, so daß sie nicht länger zum Import dieses Zukkers befähigt sind. Ein Beispiel hierfür erfolgt in *E. coli,* wo hohe intrazelluläre Fructose-1,6-bisphosphatspiegel die Phosphorylierung von HPr an Serin-46 bewirken, woraufhin dieses Molekül nicht länger am Transport eines Zuckers teilnehmen kann. Dieser intrazelluläre Zuckerspiegel, bei dem das Transportsystem abgeschaltet wird, kann zwar hinreichend sein, um die normale Funktion der Zelle aufrechtzuerhalten, ist jedoch für die Überproduktion der gewünschten Feinchemikalie einschränkend. Es ist daher wünschenswert, die erfindungsgemäßen PTS-Proteine zu modifizieren, so daß sie nicht länger für eine solche negative Regulation empfänglich sind. Dadurch werden größere intrazelluläre Konzentrationen von einem oder mehreren Zuckern, und durch Extension, eine effizientere Produktion oder größere Ausbeuten von einer oder mehreren Feinchemikalien aus Organismen, die diese mutanten PTS-Proteine enthalten, erzielt.

Diese vorstehend genannte Liste von Mutagenesestrategien für PTS-Proteine, die erhöhte Ausbeuten einer gewünschten Verbindung bewirken sollen, soll nicht einschränkend sein; Variationen dieser Mutagenesestrategien sind dem Fachmann leicht ersichtlich. Durch diese Mechanismen können die erfindungsgemäßen Nukleinsäure- und Proteinmoleküle verwendet werden, um *C*. *glutamicum* oder verwandte Bakterienstämme, die mutierte PTS-Nukleinsäure- und Proteinmoleküle exprimieren, zu erzeugen, so daß die Ausbeute, Produktion und/oder Effizienz der Produktion einer gewünschten Verbindung verbessert wird. Die gewünschte Verbindung kann ein natürliches Produkt von *C. glutamicum* sein, welches die Endprodukte der Biosynthesewege und Zwischenprodukte natürlich vorkommender metabolischer Wege sowie Moleküle umfaßt, die im Metabolismus von *C*. *glutamicum* nicht natürlich vorkommen, die jedoch von einem erfindungsgemäßen *C. glutamicum-*Stamm produziert werden.

Diese Erfindung wird durch die nachstehenden Beispiele weiter veranschaulicht, die nicht als einschränkend aufgefaßt werden sollen. Die Inhalte sämtlicher, in dieser Patentanmeldung zitierter Literaturstellen, Patentanmeldungen, Patente und veröffentlichter Patentanmeldungen sind hiermit durch Bezugnahme aufgenommen.

### Beispiele

### Beispiel 1: Präparation der gesamten genomischen DNA aus Corynebacterium glutamicum ATCC13032

Eine Kultur von *Corynebacterium glutamicum* (ATCC 13032) wurde über Nacht bei 30°C unter starkem Schütteln in BHI-Medium (Difco) gezüchtet. Die Zellen wurden durch Zentrifugation geerntet, der Überstand wurde verworfen, und die Zellen wurden in 5ml Puffer I (5% des Ursprungsvolumens der Kultur - sämtliche angegebenen Volumina sind für 100 ml Kulturvolumen berechnet) resuspendiert. Die Zusammensetzung von Puffer I: 140,34 g/l Saccharose, 2,46 g/l MgSO₄ · 7 H₂O 10 ml/l KH₂PO₄-Lösung (100g/l, mit KOH eingestellt auf pH-Wert 6,7), 50 ml/l M12-Konzentrat (10 g/l (NH₄)₂SO₄, 1 g/l NaCl, 2 g/l MgSO₄ · 7 H₂O, 0,2 g/l CaCl₂, 0,5 g/l Hefe-Extrakt (Difco), 10 ml/l Spurenelemente-Mischung (200 mg/l FeSO₄ · H₂O, 10 mg/l ZnSO₄ · 7 H₂O, 3 mg/l MnCl₂ · 4 H₂O, 30 mg/l H₃BO₃, 20 mg/l CoCl₂ · 6 H₂O, 1 mg/l NiCl₂ · 6 H₂O, 3 mg/l Na₂MoO₄ · 2 H₂O, 500 mg/l Komplexbildner (EDTA oder Citronensäure), 100 ml/l Vitamingemisch (0,2 ml/l Biotin, 0,2 mg/l Folsäure, 20 mg/l p-Aminobenzoesäure, 20 mg/l Riboflavin, 40 mg/l Ca-Panthothenat, 140 mg/l Nikotinsäure, 40 mg/l Pyridoxolhydrochlorid, 200 mg/l Myoinositol). Lysozym wurde in einer Endkonzentration von 2,5 mg/ml zur Suspension gegeben. Nach etwa 4 Std. Inkubation bei 37°C wurde die Zellwand abgebaut, und die erhaltenen Protoplasten wurden durch Zentrifugation geerntet. Das Pellet wurde einmal mit 5 ml Puffer I und einmal mit 5 ml TE-Puffer (10 mM Tris-HCl, 1 mM EDTA, pH-Wert 8) gewaschen. Das Pellet wurde in 4 ml TE-Puffer resuspendiert, und 0,5 ml SDS-Lösung (10%) und 0,5 ml NaCl-Lösung (5 M) wurden zugegeben. Nach Zugabe von Proteinase K in einer Endkonzentration von 200 µg/ml wurde die Suspension etwa 18 Std. bei 37°C inkubiert. Die DNA wurde durch Extraktion mit Phenol, Phenol-Chloroform-Isoamylalkohol und Chloroform-Isoamylalkohol mittels Standard-Verfahren gereinigt. Dann wurde die DNA durch Zugabe von 1/50 Volumen 3 M Natriumacetat und 2 Volumina Ethanol, anschließender Inkubation für 30 min bei -20°C und 30 min Zentrifugation bei 12000 U/min in einer Hochgeschwindigkeitszentrifuge mit einem SS34-Rotor (Sorvall) gefällt. Die DNA wurde in 1 ml TE-Puffer gelöst, der 20 µg/ml RNase A enthielt, und für mindestens 3 Std. bei 4°C gegen 1000 ml TE-Puffer dialysiert. Während dieser Zeit wurde der Puffer 3mal ausgetauscht. Zu Aliquots von 0,4 ml der dialysierten DNA-Lösung wurden 0,4 ml 2 M LiCl und 0,8 ml Ethanol zugegeben. Nach 30 min Inkubation bei -20°C wurde die DNA durch Zentrifugation gesammelt (13000 U/min, Biofuge Fresco, Heraeus, Hanau, Deutschland). Das DNA-Pellet wurde in TE-Puffer gelöst. Durch dieses Verfahren hergestellte DNA konnte für alle Zwecke verwendet werden, einschließlich Southern-Blotting oder zur Konstruktion genomischer Banken.

### Beispiel 2: Konstruktion genomischer Corynebacterium glutamicum (ATCC13032)-Banken in Escherichia coli

Ausgehend von DNA, hergestellt wie in Beispiel 1 beschrieben, wurden gemäß bekannter und gut eingeführter Verfahren (siehe bspw. Sambrook, J. et al. (1989) "Molecular Cloning: A Laboratory Manual". Cold Spring Harbor Laboratory Press oder Ausubel, F.M. et al. (1994) "Current Protocols in Molecular Biology", John Wiley & Sons) Cosmid- und Plasmid-Banken hergestellt.

Es ließ sich jedes Plasmid oder Cosmid einsetzen. Besondere Verwendung fanden die Plasmide pBR322 (Sutcliffe, J.G. (1979) Proc. Natl Acad. Sci. USA, 75: 3737-3741); pACYC177 (Change & Cohen (1978) J. Bacteriol. 134: 1141-1156); Plasmide der pBS-Reihe (pBSSK+, pBSSK- und andere; Stratagene, LaJolla, USA) oder Cosmide, wie SuperCos1 (Stratagene, LaJolla, USA) oder Lorist 6 (Gibson, T.J. Rosenthal, A., und Waterson, R.H. (1987) Gene 53: 283-286.

### Beispiel 3: DNA-Sequenzierung und Computer-Funktionsanalyse

Genomische Banken, wie in Beispiel 2 beschrieben, wurden zur DNA-Sequenzierung gemäß Standard-Verfahren, insbesondere dem Kettenabbruchverfahren mit ABI377-Sequenziermaschinen (s. z.B. Fleischman, R.D. et al. (1995) "Whole-genome Random Sequencing and Assembly of Haemophilus Influenzae Rd., Science 269; 496-512) verwendet. Die Sequenzierprimer mit den folgenden Nukleotid-sequenzen wurden verwendet: 5'-GGAAACAGTATGACCATG-3' oder 5'-GTAAAACGACGGCCAGT-3'.

### Beispiel 4: In-vivo-Mutagenese

In vivo-Mutagenese von *Corynebacterium glutamicum* kann durchgeführt werden, indem eine Plasmid- (oder andere Vektor-) DNA durch *E. coli* oder andere Mikroorganismen (z.B. Bacillus spp. oder Hefen, wie *Saccharomyces cerevisiae*) geleitet wird, die die Integrität ihrer genetischen Information nicht aufrechterhalten können. Übliche Mutatorstämme weisen Mutationen in den Genen für das DNA-Reparatursystem auf (z.B., mutHLS, mutD, mutT, usw., zum Vergleich siehe Rupp, W.D. (1996) DNA repair mechanisms in Escherichia coli and Salmonella, S. 2277-2294, ASM: Washington). Diese Stämme sind dem Fachmann bekannt. Die Verwendung dieser Stämme ist bspw. in Greener, A. und Callahan, M. (1994) Strategies 7; 32-34 veranschaulicht.

### Beispiel 5: DNA-Transfer zwischen Escherichia coli und Corynebacterium glutamicum

Mehrere *Corynebacterium-* und *Brevibacterium-*Arten enthalten endogene Plasmide (wie bspw. pHM1519 oder pBL1) die autonom replizieren (für einen Überblick siehe bspw. Martin, J.F. et al. (1987) Biotechnology 5: 137-146). Shuttle-Vektoren für *Escherichia coli* und *Corynebacterium glutamicum* lassen sich leicht mittels Standard-Vektoren für *E. coli* konstruieren (Sambrook, J. et al., (1989), "Molecular Cloning: A Laboratory Manual", Cold Spring Harbor Laboratory Press oder Ausubel, F.M. et al. (1994) "Current Protocols in Molecular Biology", John Wiley & Sons), denen ein Replikationsursprung für und ein geeigneter Marker aus *Corynebacterium glutamicum* beigegeben wird. Solche Replikationsursprünge werden vorzugsweise von endogenen Plasmiden entnommen, die aus *Corynebacterium-* und *Brevibactertium-*Arten isoliert worden sind. Besondere Verwendung als Transformationsmarker für diese Arten sind Gene für Kanamycin-Resistenz (wie solche, die vom Tn5- oder Tn-903-Transposon stammen) oder für Chloramphenicol (Winnacker, E.L. (1987) "From Genes to Clones - Introduction to Gene Technology, VCH, Weinheim). Es gibt zahlreiche Beispiele in der Literatur zur Herstellung einer großen Vielzahl von Shuttle-Vektoren, die in *E. coli* und *C. glutamicum* repliziert werden, und die für verschiedene Zwecke verwendet werden können, einschließlich Gen-Überexpression (siehe bspw. Yoshihama, M. et al. (1985) J. Bacteriol. 162: 591-597, Martin, J.F. et al., (1987) Biotechnology, 5: 137-146 und Eikmanns, B.J. et al. (1992) Gene 102: 93-98).

Mittels Standard-Verfahren ist es möglich, ein Gen von Interesse in einen der vorstehend beschriebenen Shuttle-Vektoren zu klonieren und solche Hybrid-Vektoren in *Corynebacterium glutamicum-*Stämme einzubringen. Die Transformation von *C. glutamicum* läßt sich durch Protoplastentransformation (Kastsumata, R. et al., (1984) J. Bacteriol. 159, 306-311), Elektroporation (Liebl, E. et al., (1989) FEMS Microbiol. Letters, 53: 399-303) und in Fällen, bei denen spezielle Vektoren verwendet werden, auch durch Konjugation erzielen (wie z.B. beschrieben in Schäfer, A., et (1990) J. Bacteriol. 172: 1663-1666). Es ist ebenfalls möglich, die Shuttle-Vektoren für *C. glutamicum* auf *E. coli* zu übertragen, indem Plasmid-DNA aus *C. glutamicum* (mittels im Fachgebiet bekannter Standard-Verfahren) präpariert wird und in *E. coli* transformiert wird. Dieser Transformationsschritt kann mit Standard-Verfahren erfolgen, jedoch wird vorteilhafterweise ein Mcr-defizienter E. coli-Stamm verwendet, wie NM522 (Gough & Murray (1983) J. Mol. Biol. 166: 1-19).

### Beispiel 6: Bestimmung der Expression des mutanten Proteins

Die Beobachtungen der Aktivität eines mutierten Proteins in einer transformierten Wirtszelle beruhen auf der Tatsache, daß das mutante Protein auf ähnliche Weise und in ähnlicher Menge exprimiert wird wie das Wildtyp-Protein. Ein geeignetes Verfahren zur Bestimmung der Transkriptionsmenge des mutanten Gens (ein Anzeichen für die mRNA-Menge, die für die Translation des Genprodukts verfügbar ist) ist die Durchführung eines Northern-Blots (s. bspw. Ausubel et al., (1988) Current Protocols in Molecular Biology, Wiley: New York), wobei ein Primer, der so ausgestaltet ist, daß er an das Gen von Interesse bindet, mit einer nachweisbaren (gewöhnlich radioaktiven oder chemilumineszierenden) Markierung versehen wird, so daß - wenn die Gesamt-RNA einer Kultur des Organismus extrahiert, auf einem Gel aufgetrennt, auf eine stabile Matrix übertragen und mit dieser Sonde inkubiert wird - die Bindung und die Quantität der Bindung der Sonde das Vorliegen und auch die Menge von mRNA für dieses Gen anzeigt. Diese Information ist ein Nachweis für das Ausmaß der Transkription des mutanten Gens. Gesamt-Zell-RNA läßt sich durch verschiedene Verfahren aus *Corynebacterium glutamicum* isolieren, die im Fachgebiet bekannt sind, wie beschrieben in Bormann, E.R. et al., (1992) Mol. Microbiol. 6: 317-326.

Zur Bestimmung des Vorliegens oder der relativen Menge von Protein, das aus dieser mRNA translatiert wird, können Standard-Techniken, wie Western-Blot, eingesetzt werden (s. bspw. Ausubel et al. (1988) "Current Protocols in Molecular Biology", Wiley, New York). Bei diesem Verfahren werden Gesamt-Zellproteine extrahiert, durch Gelelektrophorese getrennt, auf eine Matrix, wie Nitrocellulose, übertragen und mit einer Sonde, wie einem Antikörper, inkubiert, die an das gewünschte Protein spezifisch bindet. Diese Sonde ist gewöhnlich mit einer chemilumineszierenden oder kolorimetrischen Markierung versehen, die sich leicht nachweisen läßt. Das Vorliegen und die beobachtete Menge an Markierung zeigt das Vorliegen und die Menge des gesuchten Mutantenproteins in der Zelle an.

### Beispiel 7: Wachstum von genetisch verändertem Corynebacterium glutamicum - Medien und Anzuchtbedingungen

Genetisch veränderte Corynebakterien werden in synthetischen oder natürlichen Wachstumsmedien gezüchtet. Eine Anzahl unterschiedlicher Wachstumsmedien für Corynebakterian sind bekannt und leicht erhältlich (Lieb et al. (1989) Appl. Microbiol. Biotechnol. 32: 205-210; von der Osten et al. (1998) Biotechnology Letters 11: 11-16; Patent DE 4 120 867; Liebl (1992) "The Genus Corynebacterium", in: The Procaryotes, Bd. II, Balows, A., et al., Hrsg. Springer-Verlag). Diese Medien bestehen aus einer oder mehreren Kohlenstoffquellen, Stickstoffquellen, anorganischen Salzen, Vitaminen und Spurenelementen. Bevorzugte Kohlenstoffquellen sind Zucker, wie Mono-, Di- oder Polysaccharide. Sehr gute Kohlenstoffquellen sind bspw. Glucose, Fructose, Mannose, Galactose, Ribose, Sorbose, Ribulose, Lactose, Maltose, Saccharose, Raffinose, Stärke oder Cellulose. Man kann Zucker auch über komplexe Verbindungen, wie Melassen, oder andere Nebenprodukte aus der Zucker-Raffinierung zu den Medien geben. Es kann auch vorteilhaft sein, Gemische verschiedener Kohlenstoffquellen zuzugeben. Andere mögliche Kohlenstoffquellen sind Alkohole und organische Säuren, wie Methanol, Ethanol, Essigsäure oder Milchsäure. Stickstoffquellen sind gewöhnlich organische oder anorganische Stickstoffverbindungen oder Materialien, die diese Verbindungen enthalten. Beispielhafte Stickstoffquellen umfassen Ammoniak-Gas oder Ammoniumsalze, wie NH₄Cl oder (NH₄)₂SO₄, NH₄OH Nitrate, Harnstoff, Aminosäuren oder komplexe Stickstoffquellen, wie Maisquellwasser, Sojamehl, Sojaprotein, Hefeextrakte, Fleischextrakte und andere.

Anorganische Salzverbindungen, die in den Medien enthalten sein können, umfassen die Chlorid-, Phosphor-, oder Sulfatsalze von Calcium, Magnesium, Natrium, Kobalt, Molybdän, Kalium, Mangan, Zink, Kupfer und Eisen. Chelatbildner können zum Medium gegeben werden, um die Metallionen in Lösung zu halten. Besonders geeignete Chelatbildner umfassen Dihydroxyphenole, wie Catechol oder Protocatechuat oder organische Säuren, wie Citronensäure. Die Medien enthalten üblicherweise auch andere Wachstumsfaktoren, wie Vitamine oder Wachstumsförderer, zu denen bspw. Biotin, Riboflavin, Thiamin, Folsäure, Nikotinsäure, Panthothenat und Pyridoxin gehören. Wachstumsfaktoren und Salze stammen häufig von komplexen Medienkomponenten, wie Hefeextrakt, Melassen, Maisquellwasser und dergleichen. Die genaue Zusammensetzung der Medienverbindungen hängt stark vom jeweiligen Experiment ab und wird für jeden Fall individuell entschieden. Information über die Medienoptimierung ist erhältlich aus dem Lehrbuch "Applied Microbiol. Physiology, A Practical Approach" (Hrsg. P.M. Rhodes, P.F. Stanbury, IRL Press (1997) S. 53-73, ISBN 0 19 963577 3). Wachstumsmedien lassen sich auch von kommerziellen Anbietern beziehen, wie Standard 1 (Merck) oder BHI (Brain heart infusion, DIFCO) und dergleichen.

Sämtliche Medienkomponenten sind sterilisiert, entweder durch Hitze (20 min bei 1,5 bar und 121°C) oder durch Sterilfiltration. Die Komponenten können entweder zusammen oder nötigenfalls getrennt sterilisiert werden. Sämtliche Medienkomponenten können zu Beginn der Anzucht zugegen sein oder wahlfrei kontinuierlich oder chargenweise hinzugegeben werden.

Die Anzuchtbedingungen werden für jedes Experiment gesondert definiert. Die Temperatur sollte zwischen 15°C und 45°C liegen und kann während des Experimentes konstant gehalten oder verändert werden. Der pH-Wert des Mediums sollte im Bereich von 5 bis 8,5, vorzugsweise um 7,0 liegen, und kann durch Zugabe von Puffern zu den Medien aufrechterhalten werden. Ein beispielhafter Puffer für diesen Zweck ist ein Kaliumphosphatpuffer. Synthetische Puffer, wie MOPS, HEPES; ACES usw., können alternativ oder gleichzeitig verwendet werden. Der Anzucht-pH-Wert läßt sich während der Anzucht auch durch Zugabe von NaOH oder NH₄OH konstant halten. Werden komplexe Medienkomponenten, wie Hefe-Extrakt verwendet, sinkt der Bedarf an zusätzlichen Puffern, da viele komplexe Verbindungen eine hohe Pufferkapazität aufweisen. Beim Einsatz eines Fermenters für die Anzucht von Mikroorganismen kann der pH-Wert auch mit gasförmigem Ammoniak reguliert werden.

Die Inkubationsdauer liegt gewöhnlich in einem Bereich von mehreren Stunden bis zu mehreren Tagen. Diese Zeit wird so ausgewählt, daß sich die maximale Menge Produkt in der Brühe ansammelt. Die offenbarten Wachstumsexperimente können in einer Vielzahl von Behältern, wie Mikrotiterplatten, Glasröhrchen, Glaskolben oder Glas- oder Metallfermentern unterschiedlicher Größen durchgeführt werden. Zum Screening einer großen Anzahl von Klonen sollten die Mikroorganismen in Mikrotiterplatten, Glasröhrchen oder Schüttelkolben entweder mit oder ohne Schikanen gezüchtet werden. Vorzugsweise werden 100-ml-Schüttelkolben verwendet, die mit 10% (bezogen auf das Volumen) des erforderlichen Wachstumsmediums gefüllt sind. Die Kolben sollten auf einem Kreiselschüttler (Amplitude 25 mm) mit einer Geschwindigkeit im Bereich von 100-300 U/min geschüttelt werden. Verdampfungsverluste können durch Aufrechterhalten einer feuchten Atmosphäre verringert werden; alternativ sollte für die verdampfungsverluste eine mathematische Korrektur durchgeführt werden.

Werden genetisch modifizierte Klone untersucht, sollten auch ein unmodifizierter Kontrollklon oder ein Kontrollklon getestet werden, der das Basisplasmid ohne Insertion enthält. Das Medium wird auf eine OD₆₀₀ von 0,5 - 1,5 angeimpft, wobei Zellen verwendet werden, die auf Agarplatten gezüchtet wurden, wie CM-Platten (10 g/l Glucose, 2,5 g/l NaCl, 2 g/l Harnstoff, 10 g/l Polypepton, 5 g/l Hefeextrakt, 5 g/l Fleischextrakt, 22 g/l Agar pH-Wert 6,8 mit 2 M NaOH), die bei 30°C inkubiert worden sind. Das Animpfen der Medien erfolgt entweder durch Einbringen einer Kochsalzlösung von *C. glutamicum-*Zellen von CM-Platten oder durch Zugabe einer flüssigen Vorkultur dieses Bakteriums.

### Beispiel 8: In-vitro-Analyse der Funktion mutanter Proteine

Die Bestimmung der Aktivitäten und kinetischen Parameter von Enzymen ist im Fachgebiet gut bekannt. Experimente zur Bestimmung der Aktivität eines bestimmten veränderten Enzyms müssen an die spezifische Aktivität des Wildtypenzyms angepaßt werden, was innerhalb der Fähigkeiten des Fachmann liegt. Überblicke über Enzyme im Allgemeinen sowie spezifische Einzelheiten, die die Struktur, Kinetiken, Prinzipien, Verfahren, Anwendungen und Beispiele zur Bestimmung vieler Enzymaktivitäten betreffen, können bspw. in den nachstehenden Literaturstellen gefunden werden: Dixon, M., und Webb, E.C: (1979) Enzymes, Longmans, London; Fersht (1985) Enzyme Structure and Mechanism, Freeman, New York; Walsh (1979) Enzymatic Reaction Mechanisms. Freeman, San Francisco; Price, N.C., Stevens, L. (1982) Fundamentals of Enzymology. Oxford Univ. Press: Oxford; Boyer, P.D: Hrsg. (1983) The Enzymes, 3. Aufl. Academic Press, New York; Bisswanger, H. (1994) Enzymkinetik, 2. Aufl. VCH, Weinheim (ISBN 3527300325); Bergmeyer, H.U., Bergmeyer, J., Graßl, M. Hrsg. (1983-1986) Methods of Enzymatic Analysis, 3. Aufl. Bd. I-XII, Verlag Chemie: Weinheim; und Ullmann's Encyclopedia of Industrial Chemistry (1987) Bd. A9, "Enzymes", VCH, Weinheim, S. 352-363.

Die Aktivität von Proteinen, die an DNA binden, kann durch viele gut eingeführte Verfahren gemessen werden, wie DNA-Banden-Shift-Assays (die auch als Gelretardations-Assays bezeichnet werden). Die Wirkung dieser Proteine auf die Expression anderer Moleküle kann mit Reportergenassays (wie beschrieben in Kolmar, H. et al., (1995) EMBO J. 14: 3895-3904 und den darin zitierten Literaturstellen) gemessen werden. Reportergen-Testsysteme sind wohlbekannt und für Anwendungen in pro- und eukaryotischen Zellen etabliert, wobei Enzyme, wie beta-Galactosidase, Grün-Fluoreszenz-Protein und mehrere andere verwendet werden.

Die Bestimmung der Aktivität von Membran-Transportproteinen kann gemäß den Techniken, wie beschrieben in Gennis, R.B. (1989) "Pores, Channels and Transporters", in Biomembranes, Molecular Structure and Function, Springer: Heidelberg, S. 85-137; 199-234; und 270-322, erfolgen.

### Beispiel 9: Analyse des Einflusses von mutiertem Protein auf die Produktion des gewünschten Produktes

Die Wirkung der genetischen Modifikation in *C. glutamicum* auf die Produktion einer gewünschten Verbindung (wie einer Aminosäure) kann bestimmt werden, indem die modifizierten Mikroorganismen unter geeigneten Bedingungen (wie solchen, die vorstehend beschrieben sind) gezüchtet werden und das Medium und/oder die zellulären Komponenten auf die erhöhte Produktion des gewünschten Produktes (d.h. einer Aminosäure) untersucht wird. Solche Analysetechniken sind dem Fachmann wohlbekannt und umfassen Spektroskopie, Dünnschichtchromatographie, Färbeverfahren verschiedener Art, enzymatische und mikrobiologische Verfahren sowie analytische Chromatographie, wie Hochleistungs-Flüssigkeitschromatographie (s. bspw. Ullman, Encyclopedia of Industrial Chemistry, Bd. A2, S. 89-90 und S. 443-613, VCH: Weinheim (1985); Fallon, A., et al., (1987) "Applications of HPLC in Biochemistry" in: Laboratory Techniques in Biochemistry and Molecular Biology, Bd. 17; Rehm et al. (1993) Biotechnology, Bd. 3, Kapitel III: "Product recovery and purification", S. 469-714, VCH: Weinheim; Belter, P.A. et al. (1988) Bioseparations: downstream processing for Biotechnology, John Wiley and Sons; Kennedy, J.F. und Cabral, J.M.S. (1992) Recovery processes for biological Materials, John Wiley and Sons; Shaeiwitz, J.A. und Henry, J.D. (1988) Biochemical Separations, in Ullmann's Encyclopedia of Industrial Chemistry, Bd. B3; Kapitel 11, S. 1-27, VCH: Weinheim; und Dechow, F.J. (1989) Separation and purification techniques in biotechnology, Noyes Publications).

Zusätzlich zur Messung des Fermentationsendproduktes ist es ebenfalls möglich, andere Komponenten der Stoffwechselwege zu analysieren, die zur Produktion der gewünschten Verbindung verwendet werden, wie zwischen- und Nebenprodukte, um die Gesamt-Produktivität des Organismus, die Ausbeute und/oder die Effizienz der Produktion der Verbindung zu bestimmen. Die Analyseverfahren umfassen Messungen der Nährstoffmengen im Medium (bspw. Zucker, Kohlenwasserstoffe, Stickstoffquellen, Phosphat und andere Ionen), Messungen der Biomassezusammensetzung und des Wachstums, Analyse der Produktion gewöhnlicher Metabolite aus Biosynthesewegen und Messungen von Gasen, die während der Fermentation erzeugt werden. Standardverfahren für diese Messungen sind in Applied Microbial Physiology; A Practical Approach, P.M. Rhodes und P.F. Stanbury, Hrsg. IRL Press, S. 103-129; 131-163 und 165-192 (ISBN: 0199635773) und den darin angegebenen Literaturstellen beschrieben.

### Beispiel 10: Reinigung des gewünschten Produktes aus C. glutamicum-Kultur

Die Gewinnung des gewünschten Produktes aus *C. glutamicum*-Zellen oder aus dem Überstand der vorstehend beschriebenen Kultur kann durch verschiedene, im Fachgebiet bekannte Verfahren erfolgen. Wird das gewünschte Produkt von den Zellen nicht sezerniert, können die Zellen aus der Kultur durch langsame Zentrifugation geerntet werden, die Zellen können durch Standard-Techniken, wie mechanische Kraft oder Ultrabeschallung, lysiert werden. Die Zelltrümmer werden durch Zentrifugation entfernt, und die Überstandsfraktion, die die löslichen Proteine enthält, wird zur weiteren Reinigung der gewünschten Verbindung erhalten. Wird das Produkt von den *C. glutamicum-*Zellen sezerniert, werden die Zellen durch langsame Zentrifugation aus der Kultur entfernt, und die Überstandsfraktion wird zur weiteren Reinigung behalten.

Die Überstandsfraktion aus beiden Reinigungsverfahren wird einer Chromatographie mit einem geeigneten Harz unterworfen, wobei das gewünschte Molekül entweder auf dem Chromatographieharz zurückgehalten wird, viele Verunreinigungen in der Probe jedoch nicht, oder wobei die Verunreinigungen auf dem Harz zurückbleiben, die Probe hingegen nicht. Diese Chromatographieschritte können nötigenfalls wiederholt werden, wobei die gleichen oder andere Chromatographieharze verwendet werden. Der Fachmann ist in der Auswahl der geeigneten Chromatographieharze und der wirksamsten Anwendung für ein bestimmtes, zu reinigendes Molekül bewandert. Das gereinigte Produkt kann durch Filtration oder Ultrafiltration konzentriert und bei einer Temperatur aufbewahrt werden, bei der die Stabilität des Produktes maximal ist.

Im Fachgebiet sind viele Reinigungsverfahren bekannt, die nicht auf das vorhergehende Reinigungsverfahren eingeschränkt sind. Diese sind bspw. beschrieben in Bailey, J.E. & Ollis, D.F. Biochemical Engineering Fundamentals, McGraw-Hill: New York (1986).

Die Identität und Reinheit der isolierten Verbindungen kann durch Standard-Techniken des Fachgebiets bestimmt werden. Diese umfassen Hochleistungs-Flüssigkeitschromatographie (HPLC), spektroskopische Verfahren, Färbeverfahren, Dünnschichtchromatographie, NIRS, Enzymtest oder mikrobiologische Tests. Diese Analyseverfahren sind zusammengefaßt in: Patek et al. (1994) Appl. Environ. Microbiol. 60: 133-140; Malakhova et al. (1996) Biotekhnologiya 11: 27-32; und Schmidt et al. (1998) Bioprocess Engineer. 19: 67-70. Ulmann's Encyclopedia of Industrial Chemistry (1996) Bd. A27, VCH: Weinheim, S. 89-90, S. 521-540, S. 540-547, S. 559-566, 575-581 und S. 581-587; Michal, G (1999) Biochemical Pathways: An Atlas of Biochemistry and Molecular Biology, John Wiley and Sons; Fallon, A. et al. (1987) Applications of HPLC in Biochemistry in: Laboratory Techniques in Biochemistry and Molecular Biology, Bd. 17.

### Äquivalente

Der Fachmann erkennt oder kann - indem er lediglich Routineverfahren verwendet - viele Äquivalente der erfindungsgemäßen spezifischen Ausführungsformen feststellen. Diese Äquivalente sollen von den nachstehenden Patentansprüchen umfaßt sein.

### Die Angaben in Tabelle 1 sind folgendermassen zu verstehen:

In Spalte 1"DNA-ID" bezieht sich die jeweilige Zahl auf die SEQ ID NO des anhängenden Sequenzprotokolls. Eine "5" in der Spalte "DNA-ID" bedeutet demzufolge ein Verweis auf SEQ ID NO:5.

In Spalte 2"AS-ID" bezieht sich die jeweilige Zahl auf die SEQ ID NO des anhängenden Sequenzprotokolls. Eine "6" in der Spalte "AS-ID" bedeutet demzufolge ein Verweis auf SEQ ID N0:6.

In Spalte 3"Identifikation" wird eine eindeutige interne Bezeichnung für jede Sequenz aufgeführt.

In Spalte 4 "AS-POS" bezieht sich die jeweilige Zahl auf die Aminosäureposition der Polypeptidsequenz "AS-ID" in der gleichen Zeile. Eine "26" in der Spalte "AS-POS" bedeutet demzufolge die Aminosäureposition 26 der entsprechend angegebenen Polypeptidsequenz. Die Zählung der Position beginnt N-Terminal bei +1.

In Spalte 5 "AS-Wildtyp" bezeichnet der jeweilige Buchstabe die Aminosäure - dargestellt im Ein-Buchstaben-Code- an der in Spalte 4 angegebenen Position beim entsprechenden Wildtyp-Stamm.

In Spalte 6 "AS-Mutante" bezeichnet der jeweilige Buchstabe die Aminosäure - dargestellt im Ein-Buchstaben-Code- an der in Spalte 4 angegebenen Position beim entsprechenden Mutanten-Stamm.

In Spalte 7"Funktion" wird die physiologische Funktion der entsprechenden Polypeptidsequenz aufgeführt.

### Ein-Buchstaben-Code der proteinogenen Aminosäuren:

- A: Alanin
- C: Cystein
- D: Aspartat
- E: Glutamat
- F: Phenylalanin
- G: Glycin
- H: His
- I: Isoleucin
- K: Lysin
- L: Leucin
- M: Methionin
- N: Asparagin
- P: Prolin
- Q: Glutamin
- R: Arginin
- S: Serin
- T: Threonin
- V: Valin
- W: Tryptophan
- Y: Tyrosin

**Tabelle 1**

| Gene die für Phosphoenolpyruvat-Zucker-phosphotransferase Proteine codieren | | | | | | |
|---|---|---|---|---|---|---|
| **DNA ID:** | **AS ID:** | **ldentifikation:** | **AS Pos:** | **AS Wildtyp** | **AS Mutante** | **Funktion:** |
| 1 | 2 | RXA01244 | 108 | E | K | PHOSPHOENOLPYRUVATE--PROTEIN PHOSPHOTRANSFERASE |
| | | | 137 | R | C | PHOSPHOENOLPYRUVATE--PROTEIN PHOSPHOTRANSFERASE |
| | | | 166 | A | T | PHOSPHOENOLPYRUVATE--PROTEIN PHOSPHOTRANSFERASE |
| 5 | 6 | RXA01300 | 25 | A | T | PHOSPHOCARRIER PROTEIN HPR |
| 7 | 8 | RXA04358 | 227 | E | K | PUTATIVE PHOSPHOTRANSFERASE ENZYME 11, A COMPONENT SGCA (EC 2.7.1.69) |
| 9 | 10 | RXA06018 | 122 | K | E | PTS SYSTEM, FRUCTOSE-SPECIFIC IIBC COMPONENT (EC 2.7.1.69) |

## Patentansprüche

1. Isoliertes Nukleinsäuremolekül codierend für ein Polypeptid mit der in Tabelle 1 in Bezug genommenen Aminosäuresequenz ID:2, wobei das Nukleinsäuremolekül an der angegebenenen Aminosäureposition 108 eine andere proteinogene Aminosäure codiert als Glutamat.

2. Isoliertes Nukleinsäuremolekül nach Anspruch 1, wobei das Nukleinsäuremolekül an der angegebenenen Aminosäureposition 312 für Lysin codiert.

3. Ein Vektor, der wenigstens eine Nukleinsäuresequenz nach Anspruch 1 enthält.

4. Eine Wirtszelle, die mit wenigstens einem Vektor nach Anspruch 3 transfiziert ist.

5. Eine Wirtszelle nach Anspruch 4, wobei die Expression des besagten Nukleinsäuremoleküls zur Modulation der Produktion einer Feinchemikalie aus besagter Zelle führt.

6. Verfahren zur Herstellung einer Feinchemikalie welches die Kultivierung einer Zelle beinhaltet, die mit wenigstens einen Vektor nach Anspruch 3 transfiziert worden ist, so dass die Feinchemikalie produziert wird.

7. Verfahren nach Anspruch 6, **dadurch gekennzeichnet, daß** die Feinchemikalie eine Aminosäure ist.

8. Verfahren nach Anspruch 7, **dadurch gekennzeichnet, dass** die Aminosäure Lysin ist.
